# EUROPÄISCHE PATENTSCHRIFT

(11) **EP 1 668 126 B1**
(45) Veröffentlichungstag und Bekanntmachung des Hinweises auf die Patenterteilung: **13.02.2008**
(21) Anmeldenummer: 04761950.7
(22) Anmeldetag: 01.10.2004
(51) Int. Cl.: C12N 15/10

(54) **VERFAHREN ZUR IN VITRO EVOLUTION VON POLYPEPTIDEN**
METHOD FOR IN VITRO EVOLUTION OF POLYPEPTIDES
PROCEDE POUR FAIRE EVOLUER DES POLYPEPTIDES IN VITRO

(30) Priorität: 01.10.2003 CH 167103
(43) Veröffentlichungstag der Anmeldung: 14.06.2006
(73) Patentinhaber: ETH Zürich, 8092 Zürich (CH)
(72) Erfinder: BERTSCHINGER, Julian, 8046 Zürich (CH); HEINIS, Christian, CH-3270 Aarberg (CH)
(74) Vertreter: Kasche, André
(86) Internationale Anmeldenummer: PCT/CH2004/000610
(87) Internationale Veröffentlichungsnummer: WO 2005/030957

(56) Entgegenhaltungen:
- WO-A-98/37186
- WO-A-99/02671
- WO-A-02/066653
- US-A- 5 856 090
- DOI N ET AL: "STABLE: protein-DNA fusion system for screening of combinatorial protein libraries in vitro." FEBS LETTERS. 27 AUG 1999, Bd. 457, Nr. 2, 27. August 1999 (1999-08-27), Seiten 227-230, XP002312563 ISSN: 0014-5793
- SEPP A ET AL: "Microbead display by in vitro compartmentalisation: selection for binding using flow cytometry" FEBS LETTERS, ELSEVIER SCIENCE PUBLISHERS, AMSTERDAM, NL, Bd. 532, Nr. 3, 18. Dezember 2002 (2002-12-18), Seiten 455-458, XP004398450 ISSN: 0014-5793 in der Anmeldung erwähnt
- GRIFFITHS A D ET AL: "Directed evolution of an extremely fast phosphotriesterase by in vitro compartmentalization" EMBO JOURNAL, OXFORD UNIVERSITY PRESS, SURREY, GB, Bd. 22, Nr. 1, 2. Januar 2003 (2003-01-02), Seiten 24-35, XP002272486 ISSN: 0261-4189
- J. BERTSCHINGER AND D. NERI: "Covalent DNA display asw a novel tool for directed evolution of protein in vitro" PROTEIN EGINEERING, DESIGN AND SELECTION, Bd. 17, Nr. 9, 2. November 2004 (2004-11-02), Seiten 699-707, XP002312485 OXFORD UNIVERSITY PRESS, UK

## Beschreibung

Die vorliegende Erfindung betrifft ein Verfahren zur Herstellung und Zuordnung von Nukleinsäuren und der dadurch kodierten Polypeptide, das zur evolutionären Auswahl von Polypeptiden *in vitro* verwendet werden kann. Das erfindungsgemäße Verfahren ermöglicht nicht nur die Zuordnung von Nukleinsäuren zu den dadurch kodierten Polypeptiden, sondern darüber hinaus die Auswahl und Isolierung von Nukleinsäuren, die Polypeptide mit ausgewählten Eigenschaften kodieren. Des Weiteren ist die Erfindung auf die Verwendung von (Cytosin-5)-Methyltransferasen und auf die Verwendung von Fusionspolypeptiden oder damit kovalent gebundenen Nukleinsäure-Fusionspolypeptid-Komplexen in den erfindungsgemäßen Verfahren gerichtet.

Die Herstellung von Polypeptiden mit ausgewählten Eigenschaften (spezifische Bindungseigenschaften, spezifische Eigenschaften wie z.B. Katalyse, Aktivierung oder Hemmung biologischer Aktivitäten) ist von grossem kommerziellen Interesse. Polypeptide mit solchen Eigenschaften müssen aus einer sehr grossen Anzahl von Polypeptidvarianten identifiziert und isoliert werden. Ein solcher Prozess stellt letztendlich eine Imitation der natürlichen Evolution dar. In einem ersten Schritt werden üblicherweise eine grosse Anzahl von genetisch verschiedenen Polypeptidmutanten hergestellt. In einem zweiten Schritt werden diese Polypeptidmutanten nach bestimmten wünschenswerten Eigenschaften ausgewählt. Dieser Prozess zur Herstellung von Diversität und anschließender gezielter Auswahl kann beliebig oft wiederholt werden. Dabei muss aber die genetische Information (Genotyp) dem Polypeptid (Phänotyp) zugeordnet werden können, was meist durch physikalische Bindung beider zueinander ausgeführt wird.

Es sind derzeit eine Reihe von Verfahren zur Auswahl von Polypeptid-kodierenden Nukleinsäuren bekannt. Diese Verfahren nutzen unterschiedliche Strategien, um Geno- und Phänotyp einer Polypeptid-Bibliothek physikalisch miteinander zu verbinden.

Die "Phage-Display" genannte Technologie wird erfolgreich zur Selektion von Polypeptiden mit bestimmten Bindungseigenschaften benutzt (Übersicht in Clackson T. and Wells J.A. (1994) In vitro selection from protein and peptide libraries. Trends Biotechnol. 12(5) : 173-84). Dabei tragen filamentöse Phagenpartikel die Polypeptide (Phänotyp) auf ihrer Oberfläche und die genetische Information (Genotyp) im Innern. Die physikalische Verbindung zwischen der Nukleinsäure (DNA) und dem Genprodukt (Protein) findet während der Produktion der Phagenpartikel im Innern von bakteriellen Zellen statt. Es sind hierzu ähnliche Technologien bekannt, bei denen die Träger des Phäno- und Genotyps anstatt Phagenpartikel, Hefezellen (Yeast Display) oder Bakterienzellen (Bacterial Cell Display) sind. Diesen Technologien ist gemeinsam, dass zur Herstellung der Polypeptidbibliotheken die Polypeptidvarianten-kodierenden DNA-Moleküle in Zellen eingebracht werden. Die Herstellung grosser Mengen zirkulärer DNA und deren Transformation in Zellen ist allerdings sehr aufwendig. Zudem ist die Grösse der Polypeptidbibliotheken eingeschränkt. Nur mit sehr grossem Aufwand konnten Bibliotheken mit 10¹¹ Polypeptidvarianten hergestellt werden. Routinemässig werden Bibliotheken mit 10⁸ bis 10⁹ Polypeptidvarianten kloniert.

Bei einem weiteren Verfahren zur evolutionären Auswahl von Polypeptiden werden die auszuwählenden Polypeptide durch Fusion mit einem DNA-bindenden Protein, dem Lac Repressor, an die kodierenden Nukleinsäuren gebunden (Cull M.G. et al. (1992) Screening for receptor ligands using large libraries of peptides linked to the C terminus of the lac repressor. Proc. Natl. Acad. Sci. U S A. 89(5) : 1865-9). Das Repressor-Protein verbindet die Polypeptide mit den kodierenden Plasmiden durch nicht-kovalente Bindungen an eine Lac Operator-Sequenz auf dem Plasmid. Um zu gewährleisten, dass die Polypeptide an die für sie kodierenden Nukleinsäuren binden, findet die Reaktion im Innern bakterieller Zellen statt. Durch die *in vivo* Bindung von Geno- und Phänotyp ist auch bei dieser Technologie die Grösse der Polypeptid-Bibliothek eingeschränkt, da die Herstellung grosser Mengen zirkulärer DNA und die Transformation derer in Zellen sehr aufwendig ist. Die hier genutzte nicht-kovalente Bindung der Nukleinsäuren an die Polypeptide verlangt nach sehr milden Reaktionsbedingungen während der anschließenden Auswahlverfahren. Daher können bedingt durch die nicht-kovalente Bindung Polypeptide mit sehr guten Bindungseigenschaften (langlebige Komplexe mit langsamer Dissoziationskinetik (niedriger k_{off})) nicht ausgewählt werden, da während der für solche Selektionen benötigten langen Inkubationszeiten Nukleinsäure und Polypeptid dissozieren würden.

Beim so genannten "Ribosome Display" (oder auch Polysome Display)-Verfahren werden Polypeptide auf der Oberfläche von Ribosomen mit den dafür kodierenden Nukleinsäuren verbunden (Roberts R.W. (1999) Totally in vitro protein selection using mRNA-protein fusions and ribosome display. Curr. Opin. Chem. Biol. 3(3) : 268-73). Die Bindung kommt zustande, wenn die Translation der Ribonukleinsäure angehalten wird. Das entstehende Polypeptid bleibt zusammen mit der kodierenden mRNA am Ribosom gebunden. Mit diesem Verfahren wurden spezifisch an verschiedene Zielpolypeptide bindende Polypeptide (z.B. Peptide, Antikörper oder Ankyrine) isoliert. Das Verfahren hat den Vorteil, dass es vollständig *in vitro* stattfindet, wodurch grössere Polypeptid-Bibliotheken (> 10¹²) hergestellt werden können. Ein Nachteil der Ribosome Display Technologie ist die Notwendigkeit der Auswahl der Polypeptide unter besonderen Bedingungen (hohe Salzkonzentration, tiefe Temperatur), bei denen die RNA/Ribosom/Polypeptid-Komplexe stabil sind, die aber nicht notwendigerweise auch den Bedingungen des verwendeten Polypeptidauswahlverfahrens entsprechen.

Bei einem anderen Verfahren zur Verbindung von Phäno- und Genotyp wird mRNA zuerst kovalent an Puromycin gebunden, das dann an das mRNA-kodierte Polypeptid gebunden wird. Bei dem *"in vitro* Virus" genannten Verfahren wird mRNA, die am 3'-Ende eine Puromycin-Gruppe trägt, translatiert. Wenn das Ribosom das Ende des kodierenden Bereichs (Open Reading Frame) der mRNA erreicht, wird die Puromycin-Gruppe kovalent an das entstehende Polypeptid gebunden. Ein weiterer Nachteil des Verfahrens ist, dass der Genotyp durch mRNA kodiert ist. Eine mRNA kann durch sehr kleine RNAse-Verunreinigungen enzymatisch abgebaut werden. Es sind verschiedene dem '*in vitro* Virus' verwandte Verfahren bekannt, bei denen die RNA durch aufwendige Verfahren durch stabilere DNA ersetzt vvird (Roberts R.W. and Szostak JW. (1997) RNA-peptide fusions for the in vitro selection of peptides and proteins. Proc. Natl. Acad. Sci. U S A. 94(23) : 12297-302; U.S. Patent 6,281,344: Nucleic acid-protein fusion molecules and libraries).

Es wurde auch ein Verfahren zur *in vitro* Verbindung von Phäno- und Genotyp vorgeschlagen, das auf der Nicking-Eigenschaft des Replikationsinitiators des E. coli Bakteriophagen P2A basiert (FitzGerald, K. (1999) In vitro display technologies - new tools for drug discovery. Drug Discovery Today, Vol. 5, No. 6). Der Replikationsinitiator ist eine Endonuklease, die einen Strang der DNA aufbricht und dabei über eine Tyrosingruppe kovalent an das 5'-Ende der DNA gebunden wird. Da bei der bakteriellen Produktion von Proteinen die Translation schon während der Transkription erfolgt, kommt das neu entstandene P2A-Polypeptid-Fusionsprotein in Kontakt mit seiner kodierenden DNA. Die cis-Aktivität des Enzyms soll die Kopplung von Geno- und Phänotyp *in vitro* erlauben. Es sind jedoch keine Polypeptide bekannt, deren Eigenschaften durch dieses Verfahren verbessert wurden.

Ein weiteres bekanntes Verfahren zur *in vitro*-Verbindung von Phäno- und Genotyp beruht auf der nicht-kovalenten, aber hochaffinen Bindung von mRNA-Aptameren mit Tat-Proteinen des HIV1 (Fujita S. et al. (2002) Novel approach for linking genotype to phenotype in vitro by exploiting an extremely strong interaction between RNA and protein. J Med. Chem. 45(8) : 1598-606). Die Verbindung von Geno- und Phänotyp findet wie beim ,Ribosome Display' und '*in vitro* Virus'-Verfahren während der Translation *in vitro* statt. Das Verfahren hat den Nachteil, dass die Gefahr besteht, dass die Komponenten dissozieren. Zudem beruht das Verfahren auf mRNA zur Kodierung des Genotyps, die anfällig für RNAse-Abbau ist.

Ein ähnliches Verfahren basiert auf der Verbindung von Streptavidin-Polypeptid-Konjugaten mit der sie kodierenden biotinylierten Nukleinsäure in Mikrokompartimenten (Doi N. and Yanagawa H. (1999) STABLE: protein-DNA fusion system for screening of combinatorial protein libraries in vitro. FEBS Lett, 457(2) : 227-30). Um die cis-Konjugation von Geno- und Phänotyp zu gewährleisten, werden bei diesem Verfahren die Streptavidin-Polypeptid-Konjugate in wässrigen Kompartimenten einer Wasser-in-Öl Emulsion transkribiert und translatiert. Jedes Kompartiment enthält höchstens eine Nukleinsäure. Nach der Translation der Streptavidin-Polypeptid-Konjugate können diese an die kodierende biotinylierte DNA im Kompartiment binden. Die Polypeptid-Nukleinsäure-Konjugate können anschliessend aus der Emulsion extrahiert werden und einem Auswahlverfahren basierend auf den gewünschten Eigenschaften unterworfen werden. Eine Einschränkung dieses Verfahrens ist aber die ineffiziente Expression von Streptavidin in dem Transkriptions-/Translations-Mix.

Es sind weitere Verfahren zur Kopplung von Geno- und Phänotyp, die auch auf der Kompartimentierung von DNA zusammen mit einem Trankriptions/Translations-Mix in einer Wasser-in-Öl Emulsion beruhen, bekannt (Sepp A. et al. (2002) Microbead display by in vitro compartmentalisation: selection for binding using flow cytometry. FEBS Lett. 532(3) : 455-8; US Patent 6,489,103: In vitro sorting method). In einem solchen Verfahren wurden Kügelchen als Träger von Geno- und Phänotyp verwendet. Auf jedem Kügelchen wurde ein kodierendes DNA-Fragment und eine Vielzahl von Peptidsequenz-spezifischen Antikörpern befestigt. Das DNA-Fragment trägt die genetische Information für eine Peptidsequenz, die mit einem variables Polypeptid fusioniert ist. Die Kügelchen werden in separate Kompartimente einer Wasser-in-Öl-Emulsion zusammen mit einem Transkriptions-/Translations-Mix eingeschlossen. Die exprimierten Polypeptid-Peptidkonjugate werden durch die Bindung an die Antikörper auf den Kügelchen immobilisiert. Das Verfahren hat den Nachteil, dass auch hier Geno- und Phänotyp unter den Randbedingungen des Auswahlverfahrens dissozieren können. Dadurch besteht die Gefahr eines Austausches von Polypeptid-Peptid-Konjugaten zwischen verschiedenen Kügelchen und somit die einer falschen Zuordnung von Geno- zu Phänotyp.

Bei einem anderen Verfahren zur Kopplung von Geno- und Phänotyp *in vivo* werden Methylase-Polypeptid-Fusionspolypeptide an DNA gebunden (US Patent 5,856,090; DNA-methylase linking reaction). Die DNA enthält die Methylase-Erkennungssequenz 5'-GGCC-3', wobei die dritte Base (Cytidin) durch Fluorodeoxycytidin (F) ersetzt wird. Die neue Sequenz 5'-GGFC-3' dient als Suizid-Inhibitor (auch ,Mechanism based Inhibitor' genannt). Mit dieser Sequenz reagierende Methylase-Polypeptidfusionsproteine bleiben irreversibel an die DNA gebunden. Dazu wird zirkuläre DNA, die die Sequenz 5'-GGCC-3' enthält und gleichzeitig das Gen für ein Methylase-Polypeptid trägt, in bakterielle Zellen eingebracht. Zum Nährmedium dieser Zellen wird Fluordeoxycytidin hinzugegeben, das während der Replikation des Plasmids in die 5'-GGCC-3' Sequenz eingebaut wird. Methylase-Polypeptidfusionsproteine können kovalent an das Plasmid binden. Das Verfahren hat den Nachteil, dass die Anzahl an Methylase-Fusionsproteinen, die an ein Plasmid gebunden werden, nicht genau definiert werden kann. Gut exprimierende Polypeptidmutanten werden zahlreicher auf dem Plasmid immobilisiert, was dazu führt, dass eine gut exprimierende Polypeptid mutante mit mittleren Bindungseigenschaften wegen Aviditätseffekten einer schlechter exprimierenden, sehr gut bindenden Polypeptidmutante im Auswahlprozess überlegen sein kann. Zudem ist durch die *in vivo-* Bindung von Geno- und Phänotyp auch bei dieser Technologie die Grösse der Polypeptid-Bibliothek eingeschränkt.

Die internationale Patentanmeldung WO 98/37186 offenbart ein Verfahren zur Herstellung einer Proteinexpressionsbibliothek, bei dem die Proteine kovalent mit der sie kodierenden DNA verbunden sind. Die verwendeten Proteinkonjugate kodieren für einen Protein-DNA-bindenden Bereich (Protein A des P2 Phagen; P2A) und einen Displaybereich (Das zu untersuchende Protein).

Der Zusammenfassung der in der o.g. Patentanmeldung erwähnten Druckschrift von Liu Y. and Haggard-Ljungquist E., Nucleic Acid Research, 22, p. 5204-5210 (1994) ist jedoch zu entnehmen, dass das zur kovalenten Bindung der DNA verwendete gereinigte A Protein des Phagen P2 nicht an doppelsträngige ori-enthaltende DNA bindet, sondern nur an einzelsträngige ori-enthaltende DNA, was deutlicht macht, dass eine spezielle DNA-Struktur und/oder ein spezielles Protein notwendig ist, um das *ori* für das A-Protein zugänglich zu machen. Diese Einschränkung wurde auch bei anderen Proteinen mit gleicher Funktion beobachtet. Im experimentellen Teil dieser Druckschrift wird konkret festgestellt, dass das A Protein Inclusion-Körper ausbildet und kein lösliches Protein nachweisbar ist. Dieses Protein musste daher zuerst denaturiert und anschließend *in vitro* gefaltet werden. Die Expression dieses Proteins in funktioneller Form ist daher sehr ineffizient.

Die o.g. WO 98/37186 weist zudem darauf hin, dass P2A durch ssDNA zuerst aktiviert werden muss. Das hier beschriebene System war derart ineffizient, dass der gleiche Anmelder (Isogenica) in einer späteren Anmeldung (WO 04 022 74b) die vorausgegangene Anmeldung wie folgt würdigt:
"Another prior art method, covalent display technology, or CDT, is described in W098/37186. This method relies on covalent linkage of protein to DNA to retain the linkage of genotype to phenotype, through the cis action of the crosslinking protein. This method teaches that two requirements are needed for successful use of this technique. Firstly, proteins are required which interact in vitro with the DNA sequence which encodes them (cis action), and secondly, said proteins must establish a covalent linkage to their own DNA template. This method suffers from the fact that the DNA is chemically modified which can prevent the recovery and identification of the binding peptide of interest.
There remains a need for more versatile in vitro methods of constructing peptide libraries in addition to the methods described above, which can allow direct selection of binding activity, as well as for enzymatic activity, and that allow efficient production of complex peptide structures, while still allowing recovery of intact genetic material encoding the peptide of interest."

Es bleibt somit festzustellen, dass eine kovalente Verbindung von Geno- und Phänotyp bedingt durch Systemmängel keine praktische Anwendung hatte.

Für eine Verbindung von Polypeptiden mit der sie kodierenden DNA gilt somit zu beachten, dass die Bindung an die DNA spezifisch ist, und eine definierte Anzahl von Polypeptidmolekülen pro DNA Molekül gebunden werden. Letzteres ist wichtig, da bei Auswahlverfahren die Anzahl an Polypeptiden, die an ein DNA-Molekül gebunden sind, entscheidend für den Ausgang des Experiments sein kann. Werden z.B. spezifisch bindende Proteine ausgewählt, kann ein Aviditätseffekt dazu führen, dass Polypeptide mit niedriger Affinität selektiert werden, weil mehrere Polypeptide an ein DNA-Molekül gebunden sind. Dies ist nur selten wünschenswert, wenn es schwierig ist, überhaupt bindende Proteine gegen ein bestimmtes Molekül zu erhalten. In diesem Fall wird versucht, zuerst bindende Proteine mit niedriger Affinität auszuwählen, um anschliessend ausgehend von diesen hochaffine Proteine herzustellen. Die Selektion von Antikörpern mit der Phage Display Technologie hat gezeigt, dass es sehr schwierig ist, Antikörper mit hoher Affinität auszuwählen, wenn sich mehr als ein Antikörper auf der Phagenoberfläche befindet (Winter G. et al. (1994) Making antibodies by phage display technology. Ann. Rev. Immunol. 12 : 433-55).

Es ist daher die Aufgabe der vorliegenden Erfindung ein Verfahren zur Verfügung zu stellen, das die Nachteile des Standes der Technik nicht aufweist. Insbesondere soll bei einem solchen Verfahren die Anzahl der Polypeptide, die pro DNA-Molekül gebunden werden, gesteuert werden können. So kann z.B. ein Aviditätseffekt vermieden werden. Das Verfahren soll schneller und effizienter sein, z.B. kurze Inkubationszeiten haben und zeitaufwendige Zellzyklen vermeiden. Eine weitere Aufgabe ist es, eine Verbindung zwischen Genotyp- und Phänotyp zur Verfügung zu stellen, die robust genug ist, um Auswahlverfahren unter vielen und oft auch harschen Randbedingungen durchzuführen.

Des Weiteren liegt der vorliegenden Erfindung die Aufgabe zu Grunde, ein Verfahren zur Verfügung zu stellen, mit dem effizient, unkompliziert und schnell Nukleinsäuren nach Eigenschaften der kodierten Proteine ausgewählt werden können. Vorzugsweise kann mit diesem Verfahren eine Nukleinsäure nach den kodierten Proteineigenschaften nicht nur ausgewählt, sondern durch Verändern und Optimieren der Nukleinsäure in einzelnen oder mehreren Zyklen des Verfahrens evolutionär optimiert werden.

Die der Erfindung zugrundeliegenden Aufgaben werden durch das Verfahren gemäß Anspruch 1 gelöst.

Die vorliegende Erfindung betriff ein Verfahren zur Herstellung und Zuordnung von Nukleinsäuren und der dadurch kodierten Polypeptide, das folgende Schritte umfasst:
a) die Kompartimentierung von Nukleinsäuren zusammen mit einer *in vitro* Transkriptions-Translations-Mischung in einer Wasser-in-ÖI Emulsion,
b) die *in vitro* Expression der durch die Nukleinsäuren kodierten Fusionspolypeptide in den Mikrokompartimenten der Wasser-in-Öl-Emulsion, wobei jede Nukleinsäure an das Fusionspolypeptid gebunden wird, für das es kodiert,
wobei die Fusionspolypeptide jeweils mindestens einen konstanten Peptidanteil I und mindestens einen variablen Peptidanteil II umfassen und die Fusionspolypeptide über den Peptidanteil I mit der das Fusionspolypeptid kodierenden Nukleinsäure in Schritt b) kovalent verbunden werden, und wobei die Anzahl der derart gebundenen Fusionspolypeptide pro Nukleinsäure eine definierbare ganze Zahl ist.

Dieses Verfahren ermöglicht die Zuordnung und Herstellung von Nukleinsäuren mit den dadurch kodierten Polypeptiden. Eine solche Verbindung von Geno- und Phänotyp ist für Auswahlverfahren bei großen Mengen an Nukleinsäuren basierend auf den Eigenschaften der dadurch kodierten Proteine unerlässlich, da ansonsten jede Nukleinsäure und/oder jedes Protein einzeln in einem Gefäß gelagert und eingesetzt werden muss.

Es wurde überraschenderweise herausgefunden, dass die bei diesem *in vitro-*Verfahren eingesetzte kovalente Verbindung den Genotyp (Nukleinsäure) und den Phänotyp (das Protein) stabil miteinander verbindet und eine genaue Steuerung des Verhältnisses von Protein zu Nukleinsäure zulässt.

Der Begriff "definerbare ganze Zahl", wie er im Zusammenhang mit der vorliegenden Erfindung verwendet wird, bedeutet, dass die Nukteinsäuresequenz bzw. -struktur durch die Anzahl von Erkennungssequenzen für Nukleinsäure-bindende Proteine darin die genaue Anzahl der daran bindenden Fusionspolypeptide definiert, d.h. vorgibt.

Voraussetzung für das erfindungsgemäße Verfahren ist, dass die Nukleinsäure für ein Fusionspolypeptid kodiert, das jeweils mindestens einen konstanten Peptidanteil I umfasst, der an die das Fusionspeptid kodierende Nukleinsäure kovalent bindet, und das jeweils mindestens einen variablen Peptidanteil II umfasst, der zur Auswahl der gesuchten Nukleinsäuren in geeigneten Auswahlverfahren eingesetzt wird.

Die kovalente Bindung zwischen Nukleinsäure und Polypeptid gewährleistet während der Auswahl der Polypeptide die Stabilität des Komplexes unter Zeitweise harschen Randbedingungen, ohne dass die Verbindung zwischen Nukleinsäure und Polypeptid Schaden nimmt.

In einer bevorzugten Ausführungsform umfasst das Verfahren zusätzlich den Schritt des Extrahierens der in Schritt b) hergestellten Fusionspolypeptid-Nukleinsäure-Komplexe aus der Wasser-in-Öl-Emulsion.

Durch das Extrahieren der Fusionspolypeptid-Nukleinsäure-Komplexe aus der Wasser-in-ÖI-Emulsion können die Komplexe auf nachfolgende Schritte, z.B. Auswahl-/Selektionsverfahren, vorbereitet werden. Andere dem Fachmann auf dem Gebiet geläufige Reinigungs- und/oder Isolierungsverfahren können auch verwendet werden.

In einer weiteren bevorzugten Ausführungsform umfasst das erfindungsgemäße Verfahren zusätzlich den Schritt des Auswählens von solchen Fusionspolypeptid-Nukleinsäure-Komplexen, deren variabler Fusionspeptidanteil gewünschte Eigenschaften aufweist. Diese Eigenschaften können eine spezifische Bindung an andere Moleküle, z.B. Proteine, Peptide, Metalle, Polymere, etc., sein oder aber auch bestimmte biologische Funktionen wie eine katalytische Wirkung oder die Aktivierung oder Hemmung anderer Moleküle oder biologischer Systeme, z.B. zellfreier und Zell- oder auch Gewebesysteme. Vorzugsweise wird das gesamte erfindungsgemäße Verfahren *in vitro* durchgeführt. Der Auswahlschritt kann aber auch die Verwendung von beispielsweise Zellen und Geweben beinhalten. Dem Fachmann stehen als Auswahlverfahren alle auf dem Gebiet bekannte Verfahren zur Auswahl von Proteinen zur Verfügung, die ggf. routinemäßig an die Besonderheiten des jeweiligen DNA-Fusionpolypeptid-Komplexes angepasst werden können. Voraussetzung für solche Auswahlverfahren ist lediglich, dass weder Fusionspolypeptid noch DNA oder die Bindung zwischen beiden beeinträchtigt, d.h. verändert oder zerstört, wird. Als Auswahlverfahren können typische Screening-Verfahren eingesetzt werden, bei denen eine Vielzahl von Substanzen gleichzeitig und als Gesamtheit untersucht werden, aber auch Selektionsverfahren, bei denen das Ergebnis für jede Versuchssubstanz (hier DNA-Proteinkomplexe) einzeln festgestellt wird. Als Auswahlverfahren kann eines oder mehrere, gleiche oder verschiedene, parallel oder nacheinander eingesetzt werden. Beispielhafte Ausführungsformen von Auswahlverfahren werden in den Beispielen gezeigt.

Eine weitere bevorzugte Ausführungsform des erfindungsgemäßen Verfahrens umfasst ggf. nach einem vorhergehenden Extraktionsschritt das Amplifizieren der ausgewählten Nukleinsäuremoleküle. Durch das Amplifizieren wird der ausgewählten Genotyp wieder vom Phänotyp getrennt. Die amplifizierten Nukleinsäuren können nun zur Herstellung der kodierenden Proteine und Peptide eingesetzt werden oder aber auch erneut ein erfindungsgemäßen Verfahren, z.B. mit einem oder mehreren anderen Auswahlverfahren durchlaufen, um so eine Unterauswahl zu treffen.

In einer besonders bevorzugten Ausführungsform umfasst das erfindungsgemäße Verfahren zusätzlich den Schritt der zufälligen oder gerichteten Mutation der aus dem Verfahren resultierenden Nukleinsäuren. Unter Mutation ist beispielsweise das Substituieren, Deletieren, chemische Verändern oder Einfügen von einem oder mehreren Nucleotid(en) während oder nach dem Amplifizieren von Schritt e) zu verstehen. Eine zufällige oder gerichtete Mutation erlaubt es, die bereits ausgewählte Nukleinsäure bezüglich geänderter Eigenschaften neu in das erfindungsgemäße Verfahren einzusetzen und somit durch das gleiche oder verschieden Auswahlverfahren zu optimieren. Beispielsweise kann so eine Nukleinsäure, deren Proteinprodukt bereits als spezifisch bindend ausgewählt wurde, so noch weiter erfindungsgemäß optimiert werden. Auch kann der Fachmann mit dem erfindungsgemäßen Verfahren Nukleinsäuren bzw. ihre Polypeptidprodukte im Hinblick auf eine aktivierende, hemmende oder katalysierende Wirkung hin optimieren.

In einer weiteren bevorzugten Ausführungsform umfasst das erfindungsgemäße Verfahren zusätzlich den Schritt der ein- oder mehrfachen Wiederholung eines der vorangegangenen Verfahren mit dem gleichen Auswahlverfahren oder verschiedenen Auswahlverfahren zur Optimierung der ausgewählten Nukleinsäuren, ggf. nach einfacher oder mehrfacher Mutation der Nukleinsäuren.

Vorzugsweise sind die in den erfindungsgemäßen Verfahren eingesetzten Nukleinsäuren doppelsträngige rRNA, mRNA oder DNA. Besonders bevorzugt sind die Nukleinsäuren DNA und ganz besonders bevorzugt lineare DNA, weil diese besonders schnell und einfach mittels Polymerase Chain Reaction herzustellen sind.

In einer weiteren bevorzugten Ausführungsform sind die in den erfindungsgemäßen Verfahren eingesetzten Nukleinsäuren chemisch modifizierte Nukleinsäuren, insbesondere chemisch modifizierte DNA. Chemisch modifizierte DNA ist solche, die andere als die üblichen Nukleotide und/oder zusätzliche chemische Bausteine als die vier natürlich vorkommenden Basen A,T,G und C enthält. Solche Modifikation können beispielsweise für die kovalente Bindung an den konstanten Peptidanteil I des kodierten Fusionspolypeptids nützlich sein. Wenn die Modifikation nicht durch übliche Amplifikation eingeführt werden kann, kann beispielsweise die Modifikation(en) direkt vor dem Kompartimentierungsschritt a) oder im Amptifikationsschritt e) mit Hilfe entsprechend modifizierter Primer eingeführt werden. Andere chemische Verfahren zur Einführung von Modifikationen in Nukleinsäuren sind dem Fachmann bekannt und können in der vorliegenden Erfindung zum Einsatz kommen.

Vorzugsweise umfasst jedes Mikrokompartiment der Wasser-in-Öl Emulsion, die in dem erfindungsgemäßen Verfahren eingesetzt wird, nicht mehr als eine Nukleinsäure. So kann sichergestellt werden, dass die Zuordnung einer Nukleinsäure zu dem von ihr kodierten Polypeptid, d.h. die Bindung der Beiden, zu keinen Fehlinformationen bei den Auswahlverfahren führt.

Eine solche Zuordnung wird bei aus Wasser-in-Öl-Emulsion hergestellten Mikrokompartimenten meist durch solche mit einem mittleren Durchmesser von 1 µm bis 2 µm sichergestellt, wobei Mikrokompartimente dieser Größe bevorzugte Ausführungsformen der vorliegenden Erfindung darstellen.

Bei dem erfindungsgemäßen Verfahren ist vorzugsweise jeweils ein konstanter Peptidanteil I mit einem Nukleinsäuremolekül kovalent verbunden. Dieses 1 : 1 - Verhältnis vermeidet Aviditätseffekte, Ausfällen und insbesondere eine sterische Hinderung des Proteinanteils bei Auswahlverfahren, bei denen die Auswahl auch von der Zugänglichkeit der die Auswahl vermittelnden Bereiche abhängt.

In einer bevorzugten Ausführungsform ist der konstante Peptidanteil I des Fusionspolypeptids eine (Cytosin-5)-Methyltransferase.

Es hat sich überraschender Weise gezeigt, dass Methyltransferasen *in vitro* mit hoher Stabilität an Nukleinsäuren binden und zudem leicht *in vitro* transkribiert und translatiert werden. Die DNA-Bindung dieser Verbindungen hält auch unter den harschen experimentellen Bedingungen der meisten Auswahlverfahren für Proteine stand. Überraschend ist auch ihr Einsatz für lineare DNA. Bisher wurden Methyltransferasen lediglich *in vivo* in Zellen eingesetzt, um an zirkuläre Plasmide zu binden.

DNA-(Cytosin-5)-Methylasen kommen in prokaryontischen sowie eukaryontischen Organismen vor. Die Aminosäuresequenzen der Mitglieder der Familie der prokaryontischen (Cytosin-5)-Methyltransferasen weisen einen hohen Grad an Homologie auf. Am stärksten tritt diese Homologie in 10 konservierten Regionen dieser Proteine auf. Alle (Cytosin-5)-Methyltransferasen übertragen eine Methylgruppe vom Kofaktor S-Adenosylmethionin auf die Position 5 eines Cytosins in der DNA.

Vorzugsweise wird die Methylgruppe aus der Gruppe von Methyltransferasen ausgewählt, die aus M.Hae III, M.Hha I, M.Hpa I, M.Msp I, und Alu I besteht.

Im Folgenden werden die o.g. bevorzugten Methyltransferasen und ihre DNA-Erkennungssequenz gezeigt.

| | | |
|---|---|---|
| M.Hae III | *Haemophilus aegypticus* | 5'-GG**C**C-3' |
| M.Hha I | *Haemophilus heamolyticus* | 5'-G**C**GC-3' |
| M.Hpa I | *Haemophilus parainfluenzae* | 5'-**C**CGG-3' |
| M.Msp I | *Moraxella species* | 5'-**C**CGG-3' |
| Alu I | *Arthrobacter luteus* | 5'-AG**C**T-3' |

Weitere erfindungsgemäß verwendbare Methylasen sind dem Fachmann bekannt, bzw. sind leicht aufzufinden (z.B. im Katalog von *New England Biolabs,* die gereinigte Enzyme verkaufen).

Neben den zuvor genannten Methylasen können aber auch andere dem Fachmann bekannte Proteine oder Peptide erfindungsgemäß eingesetzt werden, um DNA kovalent zu binden. Vorzugsweise handelt es sich dabei um terminale Proteine.

Kovalent an DNA bindende Proteine sind beispielsweise aus Phagen von *Streptomyces pneumoniae* und *E.coli* (Bsp. Phi29, Cp-1 and PRD1) bekannt. Weitere derartige Proteine gibt es in Viren, z.B. Adenovirus, bei linearen Plasmiden (Bsp. S1, Kalilo) und auch in Bakterien (z.B. *Streptomyces).*

Das terminale Protein (TP) des Bakteriophagen phi29 ist das am besten untersuchte. Es bindet an das 5'-Ende der DNA. Bei der Replikation des Genoms von phi29 wird das Ende des neu synthetisierten DNA-Stranges mit dem terminalen Protein verbunden (Protein Priming Mechanismus). Dazu ist aber ein quaternärer Komplex aus "alter TP-DAN", phi29 DNA Polymerase und "neuem" TP notwendig. Dieses System ist allerdings in *in vitro* Expressionsystemen mit anschliessendem direktem Cross-linking nicht praktikabel. Meijer, W.J.J., Horcajadas J.A., Salas M., phi29 family of phages, Microbiology and Molecular Biology Reviews (2001), p. 261-287.

Besonders bevorzugt für die Durchführung des erfindungsgemäßen Verfahrens ist die Methyltransferase Hae III aus *Haemophilus aegypticus* ist.

Ganz besonders bevorzugt in diesem Zusammenhang ist die Verwendung einer modifizierten Nukleinsäuren, die die Sequenz 5'-GGFC-3' umfasst, wobei F 5-Fluorodeoxycytidin ist, als Erkennungssequenz der Methyltransferase.

Ein weiterer Aspekt der Erfindung betrifft die Verwendung bevorzugter Reagenzien zur Durchführung des erfindungsgemäßen Verfahrens.

Eine bevorzugte Ausführungsform ist dabei die Verwendung mindestens einer (Cytosin-5)-Methyltransferase in einem erfindungsgemäßen Verfahren.

Eine weitere bevorzugte Ausführungsform ist dabei die Verwendung von Fusionspolypeptiden oder damit kovalent gebundenen Nukleinsäure-Fusionspolypeptid-Komplexen in einem erfindungsgemäßen Verfahren, die jeweils mindestens einen konstanten Peptidanteil I und mindestens einen variablen Peptidanteil II umfassen, wobei die Fusionspolypeptide über den Peptidanteil I mit der das Fusionspolypeptid kodierenden Nukleinsäure kovalent verbunden werden oder verbunden sind, und wobei die Anzahl der derart gebundenen Fusionspolypeptide pro Nukleinsäure eine definierbare ganze Zahl ist.

Im Folgenden werden einzelne Verfahrensschritte der vorliegenden Erfindung anhand der Figuren beispielhaft erläutert.

In einem ersten Schritt **A** in Figur A wird eine Sammlung **1** von zueinander leicht unterschiedlichen Genen (DNA-Bibliothek **1)** zusammen mit einer Suspension, die die Expression dieser Gene ermöglicht (Transkriptions-/Translationslösung), in der Wasserphase einer Wasser-in-Öl-Emulsion **3A** eingeschlossen. Dies geschieht vorzugsweise derart, dass pro Wasserkompartiment 3B maximal eine Nukleinsäure (vorzugsweise ein lineares DNA Molekül **2)** vorhanden ist. Durch die Komponenten der Transkriptions-/Tranlsationslösung wird nun das in dem Wasserkompartiment vorhandene Gen als Polypeptid exprimiert.

Erfindungsgemäss hergestellte Fusionspolypeptide **5** umfassen die zwei Peptidanteile I und II. Der Peptidanteil I **5A** ist ein Polypeptid, das mit einer am DNA-Molekül vorhandenen chemischen Gruppe oder der Nukleinsäure selbst reagieren kann. Diese chemische Gruppe (Stern *, hier am linken Ende der DNA **2)** kann entweder in der Sequenz der DNA **2** angeordnet sein oder an einem der Enden der DNA **2** angefügt werden. Durch eine chemische Reaktion wird zwischen dem Polypeptid und dem DNA-Molekül eine kovalente Bindung und damit ein Polypeptid-DNA-Komplex **6** ausgebildet. Der variable Peptidanteil II **5B** ist ein Polypeptid, dessen Eigenschaften durch den erfindungsgemäßen Auswahlschritt bestimmt werden. Durch das erfindungsgemäße Verfahren findet letztendlich eine *in vitro* Evolution statt.

Die DNA-Polypeptid-Fusionskomplexe **6** werden nach erfolgter Bindung vorzugsweise von der Emulsion durch Extraktion getrennt (Schritt **B).** Man erhält so eine Sammlung **4** von DNA-Polypeptid-Komplexen **6,** in denen die DNA Moleküle **2** kovalent an die Polypeptide **5A/5B** gebunden sind, wobei jedes Nukleinsäuremolekül **2** an dasjenige Fusionspolypeptid 5 gebunden wird, für welches es kodiert.

Mit dieser Sammlung **4** von DNA-Polypeptid-Fusionskomplexen **6** werden im erfindungsgemäßen Auswahlverfahren Polypeptide mit ausgewählten, also vorbestimmten Eigenschaften ausgewählt, gescreened bzw. selektiert. (Schritt **C**). Die Auswahl von spezifisch bindenden Polypeptiden erfolgt zum Beispiel mittels Affinitätsreinigung. Dabei wird die Sammlung **4** aus Polypeptid-DNA-Komplexen **6** zu einem immobilisierten Zielmolekül **8** hinzugegeben, gegen weiches ein spezifisch bindendes Polypeptid **7** gefunden werden soll. Die nicht-bindenden Polypeptid-DNA-Komplexe werden weggewaschen.

Anschliessend (Schritt **D)** wird die genetische Information der gebundenen Polypeptide **7** durch PCR (Polymerase Chain Reaction) amplifiziert und so vom Komplex abgetrennt. Durch die Amplifikation erhält man eine neue Sammlung **9** von Genen, mit welcher ein weiterer Polypeptid-DNA-Komplex und Auswahlzyklus durchgeführt werden kann (Weg **E).** Nach einer genügenden Anzahl von solchen erfindungsgemäßen Auswahldurchgängen können die ausgewählten DNA-Fragmente entweder für weitere Zyklen neu mutiert oder zur näheren Charakterisierung der kodierten Polypeptide kloniert werden (Weg **F).**

Durch das erfindungsgemäße Verfahren kann der Prozess der Evolution, die Generierung von Diversität, Überleben der Besten durch Auswahl vorteilhafter Varianten, Vermehrung und Generierung neuer Diversität im Reagenzglas imitiert werden. Die Vorteile gegenüber existierenden Technologien umfassen beispielsweise:
a) Der gesamte Verfahren findet *in vitro* statt, d.h. die Transformation von lebenden Zellen, die einschränkend für die Grösse der Library ist, wird umgangen werden.
b) Der Polypeptid-Genotyp Komplex enthält vorzugsweise keine RNA. Somit spielt die Gefahr einer Kontaminierung mit RNAse (im Gegensatz zu anderen *in vitro* Methoden wie Ribosome Display oder mRNA Display) keine Rolle.
c) Das erfindungsgemässe Verfahren ermöglicht eine einfache Herstellung von DNA-Bibliotheken. Da nur PCRs durchgeführt werden müssen, ist weder ein Restriktionsverdau, noch eine Ligation oder eine Transformation von Zellen notwendig. Dadurch ergibt sich eine starke Verkürzung des Zeitbedarfs für die Herstellung einer Nukleinsäurebibliothek (wenige Tage statt mehrere Wochen). Mehrere Auswahl-/Evolutionszyklen können daher nacheinander und mit geringem Aufwand in relativ kurzer Zeit durchgeführt werden.
d) Es wird eine kovalente Bindung zwischen Polypeptid (Phänotyp) und DNA (Genotyp) erzeugt, die den Vorteil hat, dass nach der ggf. durchgeführten Extraktion der Polypeptid/DNA-Fusionskomplexe aus der Emulsion die Stabilität der Komplexe gewährleistet ist.
e) Vorzugsweise wird pro Nukleinsäuremolekül nur ein einziges Fusionspolypeptid gebunden. Bei einem minimalen Aviditätseffekt und einer erhöhten Empfindlichkeit wird dadurch die Auswahl/Selektion von hochaffinen Bindern (monovalentes Display) ermöglicht.

Erfindungsgemäß wird eine Wasser-in-Öl-Emulsion zur Kompartimentierung verwendet. Dazu werden viele kleine, von Öl umgebene Wasserkompartimente gebildet, die dazu dienen, eine Nukleinsäure/Gen (vorzugsweise DNA-Molekül) und dessen genetische Produkte räumlich zu verbinden. Die Kompartimentierung ermöglicht, den Genotyp des Gens mit den ausgewählten Eigenschaften des von ihm kodierten Produkts (RNA oder Polypeptid), also dem Phänotyp in Verbindung zu bringen. Die räumliche Zuordnung und Beschränkung gewährleistet die eindeutige Zuordnung durch die kovalente Bindung.

Bei der Herstellung der Wasser-in-Öl-Emulsion ist darauf zu achten, dass die Emulsion so stabil ist, dass die Gene/Nukleinsäuren und deren genetische Produkte (mRNA und Polypeptide) nicht zwischen verschiedenen Kompartimenten diffundieren können und es so zu einer Fehlzuordnung kommt. Die Wasserkompartimente dürfen also nicht miteinander verschmelzen. Die Wasser-in-Öl-Emulsion wird virzugsweise durch die Zugabe von Tensiden (z.B. Span 80, Tween 80) zur Ölphase (z.B. Mineralöl) stabilisiert. So kann eine spontane Trennung der Wasser- und Ölphase verhindert werden.

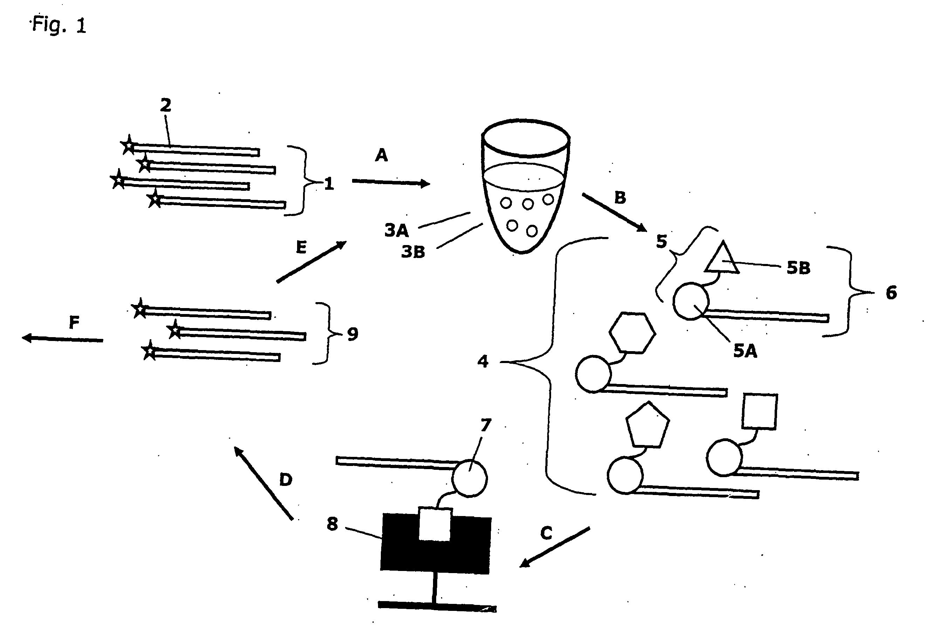

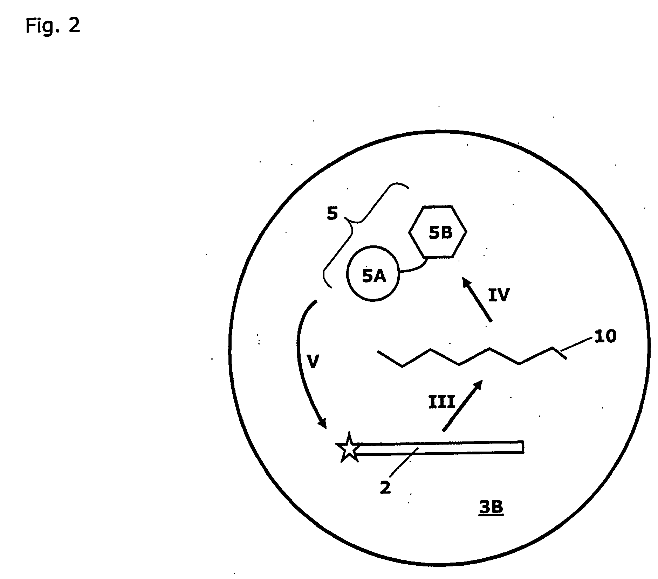

In Figur 2 werden die Prozesse innerhalb eines Mikrokompartiments bzw. Wasserkompartiments einer Wasser-in-Öl-Emulsion schematisch dargestellt. Pro Wasserkompartiment befindet sich vorzugsweise maximal ein DNA-Molekül 2 mit z.B. einem Suizid-Inhibitor (z.B. eine (Cytosin-5)-Methyltransferase-Erkennungssequenz) bzw. einer chemischen Gruppe (Sternsymbol). In einem ersten Schritt **(III,** Transkription) wird ausgehend von dem sich in dem Wasserkompartiment befindlichen DNA-Molekül **2** mRNA **10** synthetisiert, welche als Template für einen zweiten Schritt **(IV,** Translation) genutzt wird. Auf diese Weise wird das Fusionsprotein bzw. Fusionspolypeptid **5** (bestehend aus den Domänen **5A** und **5B)** exprimiert. Dieses Fusionspolypeptid **5** reagiert mit dem Suizid-Inhibitor (*) an oder auf dem DNA-Molekül (Schritt **V)** und bildet so einen DNA-Polypeptid-Komplex **6** aus (vgl. Fig. 1). Diese Verbindung von Geno- und Phänotyp ermöglicht die Auswahl/Selektion der Gene über die Eigenschaften des Phänotyps. Die anschliessende Amplifikation (hier Polymerase Chain Reaction, PCR) der ausgewählten Gene bewirkt eine Vermehrung der im Auswahlverfahren bestimmten DNA-Moleküle. Wenn nun ein Polypeptid eine kovalente Bindung mit einem DNA-Molekül eingeht, das nicht für dieses Polypeptid kodiert, dann könnten DNA-Moleküle selektiert werden, die nicht für Polypeptide mit ausgewählten Eigenschaften kodieren. Deshalb ist es bei dem erfindungsgemäßen Verfahren zur *in vitro* Evolution bedeutend, dass Polypeptide mit ihren entsprechenden Genen gekoppelt werden.

Die Grösse der Wasserkompartimente **3B** ist sehr wichtig, um einerseits die Expression der Gene (Patent US 6,489,103 B1, In vitro sorting Method) und andererseits die Bindung von DNA-Molekülen **2** mit dem exprimierten Fusionspolypeptid **5** in effizienter Weise zu gewährleisten. Die Bindungseffizienz ist von der Grösse des Wasserkompartiments abhängig, weil die Bindungsreaktion ein bimolekularer Prozess ist. Das heisst, die Geschwindigkeit der Kopplung nimmt mit steigender Konzentration der DNA und des zu koppelnden Proteins zu.

Die Konzentration der DNA gibt an, wie viele Moleküle eines Stoffes pro Volumeneinheit zu finden sind. In der vorliegenden Erfindung liegt vorzugsweise maximal ein DNA-Molekül pro Wasserkompartiment vor, weil dadurch bevorzugte Geno-Phänotyp-Fusionskomplexe erhalten werden. Aus diesem Grund sinkt die Konzentration der DNA mit der dritten Potenz mit der Zunahme des Durchmessers des Wasserkompartiments. So ergibt ein DNA-Molekül in einem Wasserkompartiment mit 2 µm Durchmesser eine Konzentration von 0.4 nM, während für ein DNA Molekül in einem Mikrokompartiment mit 1 µm Durchmesser eine Konzentration von 3.2 nM berechnet wird. Dieselben Überlegungen können für die exprimierten Polypeptide gemacht werden. Die für diese Erfindung bevorzugte Grösse (d.h. der bevorzugte Durchmesser) der Wasserkompartimente liegt zwischen 1 µm und 2 µm.

Mit einem durchschnittlichen Kompartimentdurchmesser von 1 µm können in 1 ml Emulsion etwa 10¹¹ Kompartimente gebildet werden. Es ist wünschenswert, eine möglichst hohe Zahl von Kompartimenten herzustellen, weil dann mit grösseren DNA-Bibliotheken gearbeitet werden kann. Eine gewisse Minimalgrösse der Wasserkompartimente sollte jedoch nicht unterschritten werden, weil sonst gemäss dem US Patent US 6,489,103 nicht alle Moleküle darin Platz finden würden, die für die Expression der Polypeptide benötigt werden.

Es besteht eine gewisse Toleranz des erfindungsgemässen Verfahrens in Bezug auf eine falsche positive Auswahl im ersten Auswahlzyklus. Gelangt zum Beispiel mehr als ein DNA-Fragment in ein Kompartiment, so ist es möglich, dass ein ausgewählter Phänotyp fälschlicherweise mit einem nicht erwünschten Genotyp verbunden wird. Wird der Komplex in der folgenden Auswahl isoliert, so wird dessen DNA durch die PCR Amplifikation vermehrt. Solche falsch positiv ausgewählten Genotypen stellen jedoch kein Problem dar, weil sie in einem der folgenden Auswahlzyklen eliminiert werden können.

Die Wasser-in-ÖI-Emulsion kann beispielsweise durch einfaches Vermischen der wässrigen und der organischen Phase hergestellt werden. Das Mischen kann mit verschiedenen in der Literatur beschriebenen Methoden erreicht werden (Finch C.A. et al. (1993) Encapsulation and controlled Release. Spec. Publ.-R. Soc. Chem. 138, 35). Beispielsweise kann die Ölphase mit einem Magnetrührer gerührt werden, während die wässrige Phase langsam zugetropft wird. Nach Zugabe der wässrigen Phase wird üblicherweise für eine bestimmte Zeit weiter gerührt bis die Kompartimente der Emulsion die gewünschte Grössenverteilung aufweisen. Die Rührdauer und Rührgeschwindigkeit sind sehr wichtige Parameter für die Grössenverteilung der Wasserkompartimente (Tawfik D.S. and Grffiths A.D. (1998) Man-made cell-like compartments for molecular evolution. Nat. Biotechnol. 16(7), 652).

Damit in einer Wasser-in-Öl-Emulsion Poylpeptide ausgehend von linearen oder zirkulären DNA-Fragmenten exprimiert werden können, muss zusammen mit der DNA die Maschinerie für die Proteinsynthese in die Kompartimente eingebracht werden. Diese Maschinerie besteht aus einem miteinander gekoppeltem *in vitro* Transkriptions-/Translationssystem. Es sind eine Reihe kommerzieller Produkte zu diesem Zweck erhältlich. Die zellfreie Expression von Polypeptiden in einer Wasser-in-Öl-Emulsion wurde bereits 1992 in der Literatur beschrieben (Nametkin S.N. et al. (1992) Cell-free translation in reversed micelles. FEBS 309, 330). Die Ausbeute eines in einer Wasser-in-Öl-Emulsion exprimierten Polypeptids ist gewöhnlich etwas geringer in nicht-kompartimentierter Lösung. Das Ausmass der Abnahme der Ausbeute hängt von dem exprimierten Polypeptid ab (Patent US 2002/119459, Optical sorting method).

Die Polypeptid-DNA-Komplexe können nach Expression der Polypeptide und deren Kopplung mit DNA in der wässrigen Phase aus der Emulsion extrahiert werden. Dazu wird die Emulsion zentrifugiert und die Wasserkompartimente sinken auf den Grund des Reaktionsgefässes. Die Wasserkompartimente bilden ein Sediment, sind aber immer noch intakt. Der ölige Überstand wird üblicherweise entfernt. Nun kann die wässrige Phase aus der Ölphase extrahiert werden (vgl. Tawfik D.S., and Griffiths A.D., 1998).

Vorzugsweise wird mit den extrahierten Polypeptid-DNA-Fusionskomplexen das eigentliche Auswahlexperiment durchgeführt.

Dazu kann das Molekül, für das z.B. ein bindendes Polypeptid gesucht wird, auf einer festen Oberfläche immobilisiert werden. Diese Oberfläche kann das Harz einer Chromatographiesäule, eine Plastikoberfläche oder kleine Kügelchen (Beads) sein. Die Polypeptid-DNA-Fusionskomplexe, die an das immobilisierte Molekül binden können, bleiben auch auf der festen Oberfläche, wenn das System gewaschen wird. Nach dem Waschen können die verbliebenen Polypeptid-DNA-Fusionskomplexe von der Oberfläche eluiert und anschliessend mittels PCR amplifiziert werden. Bei der Verwendung von Kügelchen können die verbliebenen DNA-Moleküle ggf. direkt nach dem Waschen (ohne Elution) amplifiziert werden. Durch die Amplifikation erhält man eine neue ausgewählte DNA-Bibliothek. Mit dieser kann entweder direkt ein weiterer Komplexbildungs- und Auswahlzyklus durchgeführt werden, oder es können neue Mutationen eingeführt werden, um die Diversität der DNA-Bibliothek zu erhöhen. Mutageneseverfahren sind in der Literatur beschrieben und dem Fachmann bekannt.

Im Folgenden wird ein bevorzugter Weg zur Ausführung der Erfindung beispielhaft beschrieben:
Für die Kopplung von Polypeptid und Nukleinsäure wird das Protein Hae III Methylase aus *Haemophilus aegypticus* (M.Hae III) (ATCC 1116) verwendet. M.Hae III methyliert in der Erkennungssequenz 5'-GGCC-3' die dritte Base von links (Cytidin, C). Ein DNA-Fragment, bei dem dieses Cytidin durch 5-fluordeoxycytidin (F) ersetzt wird (5'-GGFC-3'), dient als Suizid-Inhibitor-Erkennungssequenz (oder auch Mechanism based Inhibitor genannt) für die Hae III Methylase und ist der Ort der kovalenten Bindung zwischen DNA und Polypeptid. Dieser Suizid-Inhibitor wurde für die Aufklärung der dreidimensionalen Struktur der M.Hae III Methylase im Komplex mit ihrem Substrat entwickelt (Chen L. et al. (1991) Direct identification of the active-site nucleophile in a DNA (cytosine-5)-methyltransferase. Biochemistry 30, 11018). Durch die Verwendung von Oligonucleotiden, die die modifizierte Base 5-Fluordeoxycytidin enthalten, können die Bindungsstellen mittels PCR in die DNA, die später für die Selektionsexperimente benutzt wird, auf einfache Weise eingebaut werden. Mit 5-Fluordeoxycytidin-modifizierte Oligonucleotide sind kommerziell erhältlich (Microsynth, Balgach, Schweiz).
Das Polypeptid, das durch die erfindungsgemäße *in vitro* Evolution in seinen Eigenschaften verändert werden soll, wird an den C-Terminus der Methylase gebunden. Das Fusionsprotein besteht also aus mindestens zwei Domänen, von denen die eine (Hae III Methylase) für die kovalente Kopplung an die DNA verantwortlich ist, während die andere Domäne die auszuwählenden Eigenschaften bestimmt.
Eine DNA-Bibliothek, bestehend aus linearen DNA-Fragmenten, die für M.Hae III Fusionsproteine kodieren, wird zusammen mit Transkriptions-/Translationslösung und dem Kofaktor S-Adenosylmethionin (SAM) in eine Wasser-in-Öl-Emulsion eingebracht. Die DNA wird in den Wasserkompartimenten transkribiert und die entstandene mRNA translatiert. Auf diese Weise entstehen M.Hae III Fusionsproteine, die mit dem 5-Fluorodeoxycytidin reagieren und so eine kovalente Bindung mit der DNA eingehen. Nach Extraktion der DNA-Methylase-Fusionsprotein-Komplexe aus der Wasser-in-Öl-Emulsion kann ein Auswahl-/Selektionsexperiment durchgeführt werden, um entweder ein spezifisch bindendes oder allosterisch wirkendes Polypeptid mit ausgewählten Eigenschaften zu erhalten.

### Figuren

Die vorliegende Erfindung wird an Hand von Figuren erläutert. Dabei zeigen:
- Fig. 1: ein Schema des Auswahlzyklus gemäss der vorliegenden Erfindung;
- Fig. 2: eine schematische Darstellung der Prozesse innerhalb eines Mikrokompartiments einer Wasser-in-Öl-Emulsion;
- Fig. 3a: die Stabilität der Grössenverteilung der Wasserkompartimente der Wasser-in-Öl-Emulsion;
- Fig. 3b: den bevorzugten Durchmesser der Wasserkompartimente zwischen 1 µm und 2 µm;
- Fig. 4: die kovalente Verbindung von DNA mit M.Hae III Methylase;
- Fig. 5: die Auswahl, hier Selektion, von M.Hae III-His-tag-DANN-Komplexen mittels Ni-Affinitätschromatographie.
- Fig. 6a/b: die Auswahl, hier Selektion mittels Ni-Affinitätschromatographie, von M.Hae III-His-tag und -Flag-tag-DNA-Komplexen nach *in vitro* Expression der Polypeptide und Bildung der entsprechenden DNA-M.Hae III-Komplexe.

### Bezugszeichenliste

- A: Einschliessen der DNA-Bibliothek in Mikrokompartimente
- B: Extrahieren der Emulsion
- C: Selektieren von Polypeptiden mit den best. Eigenschaften
- D: Amplifizieren der gen Info. der geb. Polypeptide (PCR)
- E: weiterer Selektionszyklus
- F: Klonieren der kodierten Polypeptide
- III: Transkription
- IV: Translation
- 1: DNA-Bibliothek
- 2: DNA Molekül
- *: chemische Gruppe, Suicide Inhibitor
- 3A: Wasser-in-Öl Emulsion
- 3B: Wasserkompartiment
- 4: Sammlung von DNA-Polypeptid-Fusionen, Polypeptid-DNA Komplexen
- 5: Fusionspolypeptide,
- 5A: konstantes Teilpeptid I
- 5B: variables Teilpeptid II
- 6: DNA-Polypeptid-Fusion bzw. Polypeptid-DNA Komplex
- 7: gebundene Polypeptide
- 8: immobilisierte Zielmoleküle
- 9: neue Sammlung von Genen
- 10: mRNA

Im Folgenden wird die Erfindung in nicht einschränkender Weise an Hand von bevorzugten Ausführungsformen der vorliegenden Erfindung beispielhaft erläutert.

### Beispiele

### Beispiel 1

Dieses Beispiel zeigt die Herstellung einer Wasser-in-Öl-Emulsion mit vorteilhaften physikalischen Eigenschaften.

Es wurden 50 µl einer wässrigen Phase (eisgekühlter Transkriptions/Translations-Mix (Roche) mit ca. 100 ng DNA (Template für die Expression, die Menge kann variiert werden) und 80 µM S-Adenosylmethionin zu 950 µl einer eisgekühlten Ölphase (Mineralöl (Sigma, M-5904), 4.5% (v/v) Span 80 (Fluka) und 0.5% (v/v) Tween 80 (Fluka), frisch zubereitet) hinzugegeben.

Die Zugabe erfolgte tropfenweise in ein Pillengläschen (Forma Vitrum AG, 40.0 x 18.75 mm) über 2 Minuten. Während dem Zutropfen der wässrigen Phase in 5 Schritten à 10 µl wurde mit einem Magnetrührer (Heidolph MR 1000) bei 2200 Upm (Umdrehungen pro Minute) gerührt. Nach Zugabe der wässrigen Phase wurde über 5 Minuten weiter mit 2200 Upm gerührt, um die gewünschte Grössenverteilung der Kompartimente zu erreichen.

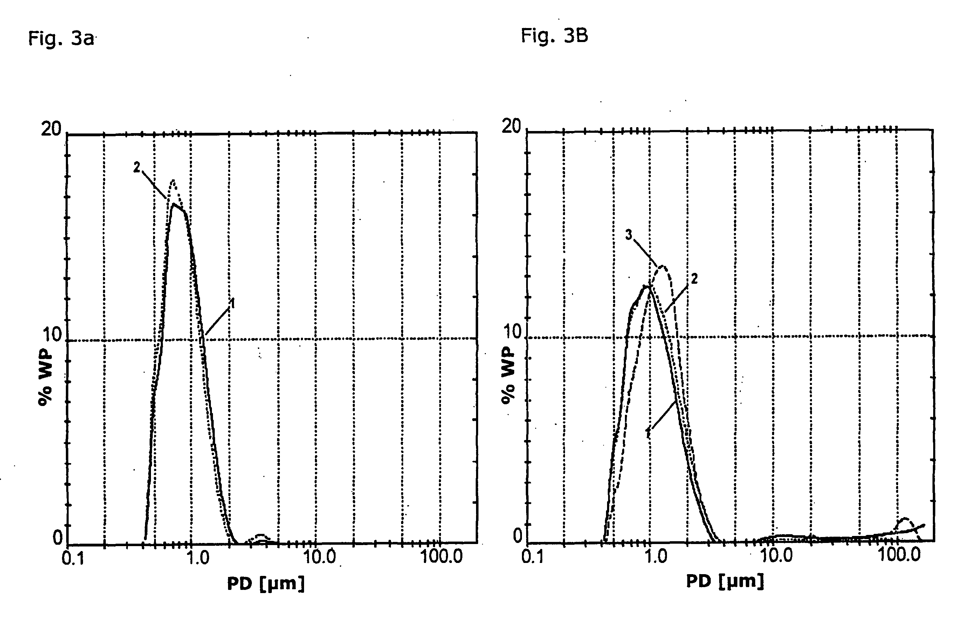

In Figur 3a und 3b werden Grössenverteilungen von Wasserkompartimenten einer Wasser-in-Öl Emulsion gezeigt, die wie oben beschrieben hergestellt wurden. Auf der X-Achse sind die Durchmesser der Mikrokompartimente (PD, in µm) in logarithmischer Skala aufgetragen. Die Werte der Y-Achse (% WP) geben den Anteil der wässrigen Phase in einem Mikrokompartiment der entsprechenden Grösse an (WP, in % des Gesamtvolumens der wässrigen Phase).

Figur 3a zeigt die Grössenverteilungen einer Wasser-in-Öl-Emulsion zu verschiedenen Zeitpunkten. Es wurde eine Emulsion hergestellt und die Grössenverteilung der Wasserkompartimente unmittelbar anschliessend mittels Lichtstreuung bestimmt (Zeit t₁ = 0 h, ausgezogene Kurve 1). Dieselbe Messung wurde noch einmal durchgeführt, nachdem die Wasser-in-Öl-Emulsion 96 Stunden bei Raumtemperatur aufbewahrt worden war (Zeit t₂ = 96 h, gestrichelte Kurve 2). Die durch diese beiden Kurven 1 und 2 dargestellten Grössenverteilungen unterscheiden sich nicht signifikant; die Emulsionen sind stabil. Die Grössenverteilungen wurden mit dem Mastersizer X (Malvern Instruments Ltd., UK) gemessen.

Figur 3b zeigt die Reproduzierbarkeit von drei Wasser-in-Öl-Emulsionen, die wie oben beschrieben hergestellt wurden. Die Profile der Grössenverteilungen (1, ausgezogene Line; 2, gepunktete Line und 3, gestrichelte Line) unterscheiden sich nicht signifikant; die Emulsionen sind reproduzierbar. Die Grössenverteilungen wurden mit dem Mastersizer X (Malvern Instruments Ltd., UK) gemessen.

### Beispiel 2

Dieses Beispiel zeigt die kovalente Verbindung on DNA einem Polypeptid.

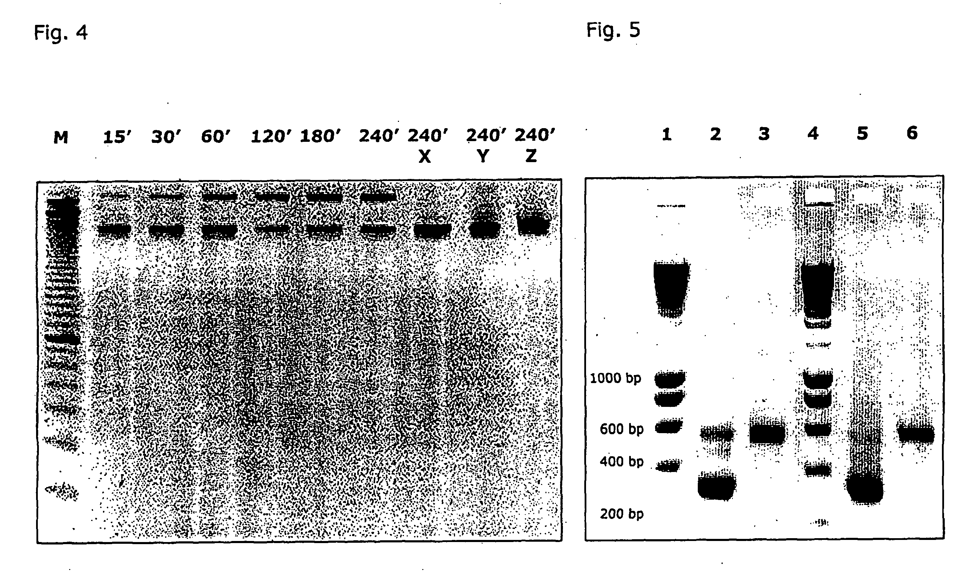

Es wurde ein 268 Bp langes DNA Fragment mit der Erkennungssequenz 5'-GGFC-3' für die hier gezeigten Kopplungsexperimente benutzt (F = 5-Fluorodeoxycytidin). 2 nM DNA wurden in Reaktionspuffer (New England Biolabs, 50 mM NaCl, 50 mM Tris-HCl (pH 8.5), 10 mM Dithiothreitol zusammen mit M.Hae III (38 nM) und 80 µM S-Adenosylmethionin (SAM) (New England Biolabs) unterschiedlich lange Zeit bei 37 °C inkubiert (15, 30, 60, 120, 180 und 240 Min.). Die Reaktionen wurden durch Erhitzen auf 70 °C für 15 Min. gestoppt (Inaktivierung der M.Hae III). Die Proben wurden auf einem denaturierenden 10% TBE Urea Gel (Novex) analysiert. Das Gel wurde mit SYBR green II (Molecular Probes, Oregon, USA) gefärbt. Auf diese Weise konnten einzelsträngige Nucleinsäuren sichtbar gemacht werden (vgl. Figur 4).

In der ersten Spur M des in Figur 4 abgebildeten Gels ist ein Grössenmarker aufgetragen (10 bp ladder, Invitrogen). In den Spuren 2 - 7 sind von links nach rechts die Proben mit zunehmender Inkubationszeit aufgetragen (über der Spur ist jeweils die Inkubationszeit von 15'-240' in Minuten angegeben). Die Spuren X, Y und Z zeigen drei Negativkontrollen:
X: Probe ohne Kofaktoren SAM;
Y: Probe ohne M.Hae III Methylase;
Z: Probe ohne das für die Reaktionen benutzte DNA Fragment (268 bp).

Der Gebrauch eines denaturierenden Gels und die vorgängige Erhitzung auf 70 °C stellen sicher, dass nur noch kovalent gebundene M.Hae III mit DNA assoziert sind. An M.Hae III gebundene DNA wandert auf dem Gel langsamer als ungebundene DNA. Es ist in Fig. 4 deutlich zu erkennen, das mit zunehmender Inkubationszeit die obere Bande an Intensität zunimmt. Das heisst, es werden mit zunehmender Inkubationszeit immer mehr DNA Moleküle an M.Hae III gebunden.

Nach ca. 2 h sind die Intensitäten der oberen und unteren Bande etwa gleich stark.

In einem doppelsträngigen DNA Molekül enthält nur ein Strang den Suizid-Inhibitor. Wenn an jeder Erkennungssequenz 5'-GGFC-3' - und daher an jeder Doppelstrang DNA ein M.Hae III kovalent gebunden ist, dann ist die Hälfte aller DNA Einzelstränge mit der Methylase verbunden. Da die obere und die untere Bande auf dem Gel dieselbe Intensität zeigen, gibt es gleich viele nicht modifizierte wie mit M.Hae III assozierte DNA Einzelstränge. Dies bedeutet, dass die Verbinundung nach ca. 2 h quantitativ erfolgte.

### Beispiel 3

In diesem Beispiel werden M.Hae III Fusionsproteine *in vitro* exprimiert.

Für die Expression von M.Hae III Fusionsproteinen wurde ein kommerziell erhältliches Trasnkriptions-/Translationssystem benutzt (RTS E.coli HY Kit, Roche Applied Science, Schweiz). Damit ein Gen mit diesem *in vitro* System exprimiert werden kann, müssen am 5'- und am 3'-Ende regulatorische DNA Sequenzen angehängt werden. Dies geschieht mittels überlappender PCR (PCR assembly). Die Sequenzen sind kommerziell erhältlich (RTS E.coli Linear Template Generation Set, His-tag, Roche Applied Science, Schweiz).

Um den Suizid-Inhibitor 5'-GGFC-3' mittels PCR in die DNA einzuführen, wurde mit den durch das Linear Template Generation Set erhaltenen DNA Fragmenten eine weitere PCR durchgeführt. Als Primer (Oligonucleotide) wurden Lin ext ba und Hae sub fo verwendet. Hae sub fo besitzt eine Erkennungssequenz für die Hae III Methylase mit einem 5-Fluorodeoxycytidin (Suicide Inhibitor). Die PCR wurde mit folgendem Temperaturprogramm ausgeführt:
94 °C (3 Min.) **→** [94 °C (1 Min.) **→** 58 °C (1 Min.) **→** 72 °C (3 Min.)]₃₀zyklen → 72 °C (5 Min.) → 4 °C.

Die Produkte der PCR wurden mit dem QIAquick PCR Purification Kit von Qiagen gereinigt.

*Sequenz von Lin ext ba:*
5'- GAT GCC GGC CAC GAT GCG TCC GGC -3'

*Sequenz von Hae sub fo:*
5'- C GTC AT**G GFC** TAT GCG GGC GAC CAC ACC CGT CCT GTG GAT -3'

DNA Templates, die für M.Hae III-His tag, M.Hae III-Flag tag, M.Hae III-Calmodulin-His tag und M.Hae III-ED-B-His tag kodieren, wurden auf dieselbe Weise hergestellt (ED-B: Extra Domain B von Fibronectin). Die Fusionen zu Hae III Methylase wurden alle an dessen C-Terminus angehängt. Die Fusionsproteine wurden in freier Lösung und in Emulsion exprimiert.

### Expression in freier Lösung:

200 ng jedes DNA Templates wurden in 25 µl *in vitro* Transkriptions-/Translationsmix (Roche Applied Science) für 3 h bei 30 °C inkubiert.

### Expression in Emulsion:

300 ng jedes DNA Templates wurden in 50 µl eisgekühlten *in vitro* Transkriptions-/Translationsmix (Roche Applied Science) gegeben. Die Wasser-in-Öl-Emulsionen wurden wie oben beschrieben zubereitet. Die fertigen Emulsionen wurden für 3 h bei 30 °C inkubiert. Nach Expression der Polypeptide und der Bildung der DNA-Polypeptid-Fusionskomplexen wurde die wässrige Phase aus der Emulsion extrahiert. Die Emulsionen wurden über 6 Minuten bei 10'000 Upm zentrifugiert, der Öl-Überstand abgesogen und 150 µl PBS zur sedimentierten Emulsion gegeben.

Dann wurde 1 ml eiskalter, wassergesättigter Diethylether zugegeben, und die Probe mit dem Vortex gut gemischt. Das Reaktionsgefäss wurde stehen gelassen, damit sich organische und wässrige Phase trennen konnten. Die wässrige Phase wurde dann unter der organischen Phase mit einer Pipette entfernt, in ein separates Reaktionsgefäss gefüllt und für 10 Minuten bei 40 °C inkubiert, um Reste des Diethylethers verdunsten zu lassen.

Die Expressionsmenge wurde mittels Western Blot analysiert (Detektion: anti-His-HRP Konjugat (Sigma) oder: anti-Flag (Sigma) mit anti-Maus-HRP Konjugat (Sigma)). Dabei zeigte es sich, dass in Emulsion etwa 20 % der in freier Lösung erwarteten Expressionsausbeute erzielt wurde. Einzig das M.Hae III-Calmodulin-His tag Fusionsprotein konnte in der Expression in Emulsion nicht nachgewiesen werden. Es wurden keine Fragmente der Fusionsproteine nachgewiesen, was auf eine niedrige Proteaseaktivität schliessen lässt.

Die Methylaseaktivität der exprimierten Fusionsproteine wurde ebenfalls analysiert. Durch Methylierung der Zielsequenz 5'-GC**GGCC**GC-3' kann ein DNA Fragment vor dem Verdau mit dem Restriktionsenzym Not I geschützt werden. Wenn ein eine Not I Schnittstelle enthaltendes DNA Fragment mit M.Hae III Fusionsproteinen inkubiert wird, kann es danach nicht mehr von Not I geschnitten werden.

Transkriptions-/Translationslösungen, in denen je ein M.Hae III Fusionsprotein exprimiert worden war, wurden mit einem eine Not I enthaltenden DNA Fragment inkubiert. Danach wurde untersucht, ob die DNA Fragmente noch mit Not I geschnitten werden können. In allen untersuchten Fällen waren die exprimierten Proteine aktiv. Eine Ausnahme bildete wiederum das in Wasser-in-Öl exprimierte M.Hae III-Calmodulin-His tag Fusionsprotein, das die DNA mit der Not I Schnittstelle nur etwa zur Hälfte schützte. Dies lässt auf ein tiefes Expressionslevel schliessen.

### Beispiel 4

Dieses Beispiel zeigt die Auswahl, hier Selektion eines DNA Fragments mittels Nickel-Affinitätschromatographie, das an M.Hae III-His tag gebunden ist. Wenn dasselbe DNA Fragment hingegen nicht an ein M.Hae III-His tag Protein gekoppelt ist, wird es nicht selektiert.

Zuerst wurde DNA mit dem M.Hae III-His tag Protein gekoppelt, indem ein für M.Hae III kodierendes DNA Template mit rekombinant hergestelltem M.Hae III-His tag inkubiert wurde.

2 nM DNA wurden in Reaktionsbuffer (New England Biolabs, 50 mM NaCl, 50 mM Tris-HCl (pH 8.5), 10 mM Dithiothreitol) zusammen mit 350 ng M.Hae III-His tag und 80 µM S-Adenosylmethionin während eineinhalb Stunden bei 37 °C inkubiert (gesamtes Reaktionsvolumen: 30 µl). Bei der Negativkontrolle wurde M.Hae III-His tag weggelassen.

Nach der Inkubation wurden 50 µl Puffer A (50 mM NaH₂PO₄, 300 mM NaCl, 10 mM Imidazol, 0.1% Tween 20 (Fluka) pH = 8.0) hinzugegeben.

20 µl Ni-NTA Magnetic Agarose Beads (Qiagen, Cat. No. 36111) wurden zugegeben und die Probe wurde für 1 Stunde bei Raumtemperatur inkubiert.

Die magnetischen Ni-NTA Agarose-Beads wurden mit Hilfe eines Magnetic Separators (MPC-S, Dynal, Norway) viermal mit 100 µl Puffer B (50 mM NaH₂PO₄, 300 mM NaCl, 20 mM Imidazol, 0.1% Tween 20, pH 8.0) gewaschen.

Nach dem letzten Waschgang wurden die Ni-NTA Magnetic Agarose Beads in 100 µl sterilem Wasser resuspendiert.

Mit 1 µl der gewaschenen Nickel-Beads wurde die Menge der verbliebenen DNA mittels quantitativer PCR analysiert (Wang A.M. et al. (1989) Quantitation of mRNA by the polymerase chain reaction. Proc. Natl. Acad. Sci. 86, 9717). In dieser PCR wurden nur die letzten 331 Basenpaare am 3'-Ende des Templates amplifiziert. Als Primer wurden die Oligonucleotide Hae end ba (downstream) und Hae sub fo short 2 (upstream) benutzt. Als Kompetitor-DNA wurde das Template eingesetzt (0.1 pM), das für das M.Hae III-ED-B-His tag Fusionsprotein kodiert. Mit den obigen Primern wird ausgehend von diesem Template ein 577 Bp langes DNA Fragment amplifiziert. Nach der Amplifikation der selektierten Nukleinsäuren wurden die Proben auf ein Agarosegel (1.4 %) aufgetragen.

Das Agarosegel wird in Figur 5 gezeigt. In Spur 1 und 4 ist ein Grössenmarker geladen (Smart Ladder, Eurogentech). Die Bande knapp unter der 600 Bp Markierung ist das DNA Fragment, das als Kompetitor zur PCR gegeben wurde. Die untere Bande ist das 331 Bp lange DNA Fragment, das mit dem Enzym M.Hae III-His tag inkubiert worden war. In Spur 2 wurde das Experiment aufgetragen, in Spur 3 die Negativkontrolle ohne M.Hae-His tag. In Spur 5 wurde als quantitativer Vergleich 0.1 pM des 331 Bp DNA Moleküls zur PCR Lösung hinzugegeben. Spur 6 zeigt das Ergebnis der PCR nur mit Kompetitor-DNA (Negativkontrolle).

### Beispiel 5

Expression von M.Hae-His tag und M.Hae-Flag tag Fusionspolypeptiden *in vitro* und anschliessende Auswahl, hier Selektion mittels Affinitätschromatographie.

Die Gene kodierend für M.Hae III-His tag (I) und M.Hae III-Flag (II) tag wurden gemäss einem dem Fachmann gängigen Verfahren in das Plasmid pIVEX 2.3d (Roche Applied Science, Schweiz) kloniert. Je 500 ng beider Plasmide wurden in 25 µl Transkriptions-/Translations-Mix (Roche Applied Science, Schweiz) über 2 h bei 30 °C inkubiert. Zusätzlich wurden lineare DNA Template (je 50 ng), die für M.Hae III-His tag (III) und M.Hae III-Flag tag (IV) kodieren, ebenfalls in 25 µl Transkriptions-/Translations-Mix über 2 h bei 30 °C inkubiert. Die Expression der Polypeptide wurde mittels Western Blot überprüft (siehe auch Beispiel 3).

Zu den Proben I bis IV wurden 50 µl Puffer A (50 mM NaH₂PO₄, 300 mM NaCI, 10 mM Imidazol, 0.1 % Tween 20 (Fluka) pH 8.0) hinzugegeben. 20 µl Ni-NTA Magnetic Agarose Beads (Qiagen, Cat. No. 36111) wurden hinzugegeben und die Proben wurden für 1 Stunde bei Raumtemperatur inkubiert. Die magnetischen Ni-NTA Agarose-Beads wurden mit Hilfe eines Magnetic Separators (MPC-S, Dynal, Norway) sechsmal mit 100 µl Puffer B (50 mM NaH₂PO₄, 300 mM NaCl, 20 mM Imidazol, 0.1 % Tween 20, pH 8.0) gewaschen. Nach dem letzten Waschgang wurden die Ni-NTA Magnetic Agarose-Beads in 100 µl PBS resuspendiert. Mit 1 µl der gewaschenen Nickel-Beads wurde die Menge der verbliebenen mittels PCR analysiert.

Für die PCR wurden die Primer M.Hae Nco ba (downstream) und M.Hae Xho His fo (upstream) benutzt. Mit diesen Primern wird ein DNA Fragment von 1020 Bp amplifiziert. Für die PCR wurde folgendes Temperaturprogramm benutzt:
94 °C (3 Min.) → [94 °C (1 Min.) → 55°C (1 Min.) → 72 °C (90 Sek.)]₂₅ Zyklen **→** 72°C (3 Min.) → 4 °C.

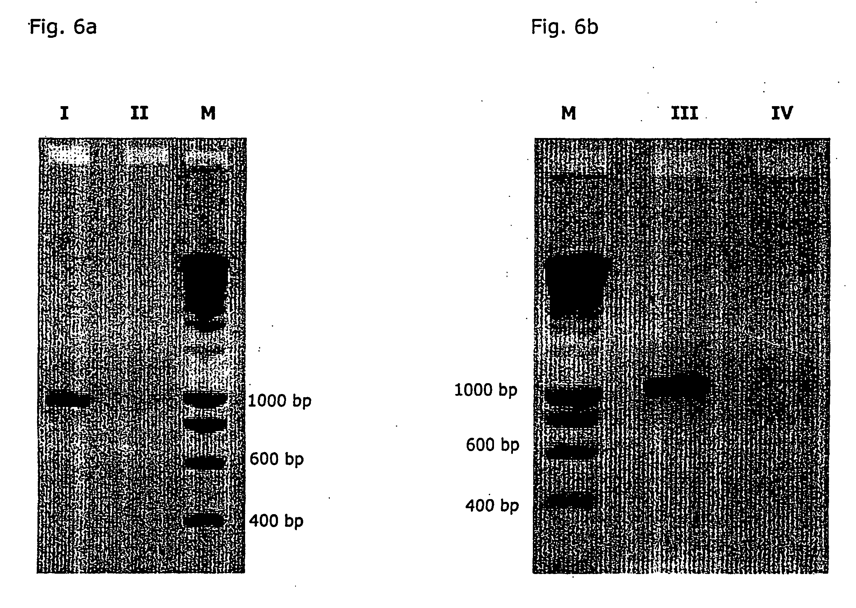

Die PCR Proben I bis IV wurden zur Analyse auf ein Agarosegel (1.4 %) aufgetragen (vgl. Figur 6a und 6b).

Figur 6a zeigt das Selektionsexperiment mit der Plasmid-DNA als Template für die *in vitro* Transkription-/Translation. In der Spur ganz rechts (M) wurden 5 µl eines Grössenmarkers geladen (Smart Ladder, Eurogentech). In der ersten Spur (I) von links wurde die Probe geladen, in der das Plasmid, das für M.Hae III-His tag kodiert, für die *in vitro* Transkription-/Translation eingesetzt worden war. In der mittleren Spur (II) wurde die Probe geladen, in der das Plasmid, das für M.Hae III-Flag tag kodiert, für die *in vitro* Transkription-/Translation eingesetzt worden war.

Figur 6b zeigt das Auswahl-, hier Selektionsexperiment mit der linearen DNA als Template für die *in vitro* Transkription-/Translation. In der Spur ganz links (M) wurden 5 µl eines Grössenmarkers geladen (Smart Ladder, Eurogentech). In der mittleren Spur (III) wurde die Probe geladen, in der die lineare DNA, die für M.Hae III-His tag kodiert, für die *in vitro* Transkription-/Translation eingesetzt worden war. In der rechten Spur (IV) wurde die Probe geladen, in der die lineare DNA, die für M.Hae III-Flag tag kodiert, für die *in vitro* Transkription-/Translation eingesetzt worden war.

Aus den Intensitäten der DNA-Banden auf dem Agarosegel von Fig. 6a und Fig. 6b ist klar zu erkennen, dass mit M.Hae III-His tag-Fusionspolypeptid gekoppelte DNA mit Nickel-Affinitätschromatographie selektiert und amplifiziert wurde, während mit M.Hae III-Flag tag-Fusionspolypeptid gebundene DNA Selektionszyklus nicht überlebte.

### Beispiel 6

Im Folgenden werden beispielhaft lineare DNA-Moleküle aufgrund der Bindungseigenschaften der von ihnen kodierten Proteine selektioniert.

Dazu wurde ein DNA-Templat hergestellt, das für das Fusionsprotein M.Hae III-Calmodulin kodiert. Dieses DNA Templat wurde auf dieselbe Weise wie in Beispiel 3 hergestellt.

Ein in vitro Transkriptions-/Translationsmix wurde gemäss den Vorschriften des Herstellers des Kits (RTS E.coli HY Kit, Roche Applied Sciences) auf Eis gekühlt zubereitet. 40 µl Transkriptions-/Translationsmix, 5 µl S-Adenosylmethionin (Endkonzentration 80 µM), 100 ng M.Hae III-Calmodulin DNA Templat (ca. 5x10¹⁰ Moleküle) und Wasser wurden so zusammengegeben, dass ein Volumen von insgesamt 50 µl erreicht wurde. Die DNA wurde erst kurz vor dem Emulgieren zugegeben. Für das Zubereiten der Emulsion wurde die wässrige Phase schrittweise (5 x 10 µl während 2 Min.) zu 950 µl der Oelphase hinzugegeben, wie es in Beispiel 1 beschrieben wird.

Für die Expression der Proteine und die Herstellung der kovalenten Protein-DNA Komplexe wurden die Proben bei 30 °C über 150 Min. inkubiert. Danach wurde die Wasserphase, welche die DNA-Protein Fusionen enthält, folgendermassen aus der Emulsion extrahiert. Die Proben wurden über 10 Min. bei 7000 Umdrehungen pro Minute zentrifugiert, wonach die Wasserkompartimente am Boden des Reaktionsgefässes sedimentierten. Der Ueberstand (Oelphase) wurde abgesaugt und 150 µl Puffer hinzugegeben (Puffer bestehend aus: TBS (Tris-gepufferte Salzlösung) mit 1 mM CaCl₂ (=TBSC), pH 7.4, 5 µM biotinylierte, doppelsträngige DNA Fragmente zur Blockierung der später verwendeten magnetischen Kügelchen [5'-Biotin-GGA GCT TCT GCA TTC TGT GTG CTG-3' (Qiagen)], 1 µM kompetitier ende, doppelsträngige DNA Fragmente [5'-ATC TAA **GGC C**AA TGT ACT AGA C**GG CC**A TTC CAG ATG CA**G GCC** AAG CGT ACA TAC **GGC C**TA GCT AAA TCA A**GG CC**G TAT CGT-3', Substratsequenz für M.Hae III fett gedruckt, (Qiagen)]) gefolgt von 1 ml Diethylether. Danach wurden die Probe mit einem Vortex für 2x 10 Sek. geschüttelt. Nach erfolgter Trennung von Wasser- und Oelphase wurde die unten liegende wässrige Phase mit einer Pipette entfernt und in einer 24-Lochplatte über 10 Min. getrocknet, damit der verbliebene Diethylether vollständig verdampfen konnte.

Während der Extraktion der Wasserphase waren je 25 µl mit Streptavidin beschichtete magnetische Kügelchen (Dynabeads, Dynal, Norwegen) mit biotinyliertem, Calmodulin-bindendem Peptid (400 nM, biotin-CAAARWKKAFIAVSAANRFKKIS (Montigiani et al., 1996)) oder mit biotinyliertem anti-Flag Antikörper M2 (2 µl/50 µl Kügelchen, M2 Antikörper, Sigma-Aldrich) für 15 Min. inkubiert worden. Das Calmodulin-bindende Peptid wurde verwendet, um die in der Wasserphase der Emulsion befindlichen M.Hae III-Calmodulin-DNA Fusionen zu selektionieren, während der anti-Flag Antikörper als Negativkontrolle benutzt wurde. Nach der Inkubation der magnetischen Kügelchen mit Peptiden bzw. Antikörper wurden sie einmal mit TBSC 0.1% Tween 20 (Fluka) gewaschen. Anschliessend wurden die Kügelchen für 15 Min. bei Raumtemperatur mit biotinylierten DNA Fragmenten (5 µM) blockiert [(5'-biotin-GGA GCT TCT -GCA TTC TGT GTG CTG-3' (Qiagen)].

Die extrahierte Wasserphase wurde in zwei Hälften geteilt und mit den wie oben beschrieben vorbereiteten magnetischen Kügelchen gemischt. Die eine Hälfte der Wasserphase wurde zu mit Calmodulin-bindenden Peptiden beschichteten Kügelchen gegeben, während die zweite Hälfte mit anti-Flag Antikörper beschichteten Kügelchen inkubiert wurde. Die beiden Proben wurden für 45 Min. bei Raumtemperatur inkubiert und alle 10 Min. sanft geschüttelt.

Die magnetischen Kügelchen wurden anschliessend mit Hilfe eines Magnetic Separators (Dynal, Norwegen) sechs mal mit je 100 µl TBSC 0.1% Tween 20 (Fluka) und einmal mit 100 µl TBSC gewaschen, um nicht bindende DNA-Protein Fusionen von der Oberfläche der magnetischen Kügelchen zu entfernen. Nach dem Waschen wurden die magnetischen Kügelchen in 100 µl Wasser aufgeschwemmt.

Es wurde nun untersucht, wie viele DNA-M.Hae III-Calmodulin-Fusionsproteine durch Bindung an die mit Calmodulin-bindenden Peptiden oder die mit anti-Flag Antikörper beschichteten magnetischen Kügelchen selektioniert worden waren. Diese Analyse wurde mit dem dem Fachmann gängigen Verfahren der "Real-time Polymerase Chain Reaction" (Real-time PCR) durchgeführt (mit dem TaqMan^{™} System von Applied Biosystems). Als Template für die Real-time PCR wurden 0.1 µl der insgesamt 100 µl im Wasser schwimmenden magnetischen Kügelchen verwendet. Jede Probe wurde dreimal gemessen.

In der Probe, bei der mit Calmodulin-bindenden Peptiden beschichtete magnetische Kügelchen für die Selektion verwendet worden waren, konnten auf den 0.1 µl Kügelchen 7.8(±1.1) x 10⁵ DNA Moleküle nachgewiesen werden. In der Negativkontrolle mit anti-Flag Antikörper wurden hingegen nur 6.9(±1.4) x 10² DNA Moleküle gemessen (in Klammern die Standarabweichungen der Messsungen). Es wurden also 1130 mal mehr DNA Moleküle, die für M.Hae III-Calmodulin kodieren, selektioniert, wenn die magnetischen Kügelchen anstatt mit anti-Flag Antikörper mit Calmodulin-bindendem Peptide beschichtet worden waren. Derselbe Versuch wurde auch mit anderen M.Hae III Fusionsproteinen (mit den entsprechenden Antikörpern auf den magnetischen Kügelchen) durchgeführt, und es wurden ähnliche Ergebnisse erzielt. Das Verhältnis (Experiment/Negativkontrolle) der Anzahl selektionierter DNA Moleküle schwankte dabei zwischen 557 und 6897.

### Beispiel 7

Um mit Bibilotheken mit veränderten (z.B. durch Hinzufügen, Substituieren, Deletieren) DNA-Molekülen arbeiten zu können, ist es mit dem hierin beschriebenen Verfahren möglich, nur solche Protein-DNA-Fusionenkonjugate aus der Bibliothek auszuwählen, die die gewünschten Bindungseigenschaften besitzen.

Daher wurden Modellexperimente mit Mischungen bestehend aus zwei verschiedenen DNA Templaten durchgeführt. Das eine Templat kodierte für das Fusionsprotein M.Hae III-Calmodulin, das andere für M.Hae III-ED-B. Die Template wurden auf dieselbe Weise wie in Beispiel 3 beschrieben hergestellt. Das Experiment wurde, wenn nicht anders beschrieben, gemäss dem Protokoll des Beispiels 6 durchgeführt.

Zum Transkriptions-/Translationsmix wurde eine Mischung mit insgesamt 10⁹ DNA Molekülen hinzugegeben, in welcher 4200 mal mehr DNA Moleküle das Fusionsprotein M.Hae III-ED-B als M.Hae III-Calmodulin kodierten. Das Selektionsexperiment wurde mit magnetischen Kügelchen durchgeführt, die entweder mit Calmodulin-bindenden Peptiden oder mit anti-Flag Antikörpern (M2, Sigma-Aldrich) beschichtet worden waren. Der Ausgang des Experimentes wurde mittels Real-time PCR ausgewertet. Die magnetischen Kügelchen wurden aber nicht direkt für die Real-time PCR verwendet, sondern die selektionierten DNA Moleküle wurden zuerst in einer PCR mit den Primern Ampl ba (5'-CCC GCG AAA TTA ATA CGA CTC A-3', Qiagen) and Ampl fo (5'-AAA ACC CCT CAA GAC CCG TT-3', Qiagen) amplifiziert. Die PCR wurde mit folgendem Temperaturprogramm ausgeführt:
94 °C (3 Min.) **→** [94 °C (45 Sek.) → 51 °C (1 Min.)
→ 72 °C (100 Sek.)]_{35 Zyklen} **→** 72 °C (3 Min.) → 4 °C.

Das Verhältnis von DNA-Molekülen kodierend für M.Hae III-ED-B und M.Hae III-Calmodulin wurde nach Verdünnung der Proben von 1:10⁵ in Wasser mit Real-time PCR gemessen [mit TaqMan^{™} Proben spezifisch für die Gene von ED-B oder Calmodulin (Microsynth, Balgach, Schweiz)]. Es wurde 1 µl der verdünnten DNA Lösungen für die Messungen benutzt, wobei jede Messung dreimal durchgeführt wurde. Im Fall der Negativkontrolle (anti-Flag Antikörper auf den magnetische Kügelchen) konnten keine DNA-Moleküle nachgewiesen werden, die für M.Hae III-Calmodulin kodieren. In der Probe, bei der magnetische Kügelchen mit Calmodulin-bindenden Peptiden verwendet worden waren, wurden 1.4(±0.2)x10⁶ DNA Moleküle kodierend für M.Hae III-ED-B und 5.1(±0.7)x10⁴ DNA Moleküle kodierend für M.Hae III-Calmodulin nachgewiesen (in Klammern die Standardabweichung der Messungen). Das Verhältnis von M.Hae III-ED-B zu M.Hae III-Calmodulin betrug demzufolge nach der Selektion 27. Durch Vergleichen der Verhältnisse der DNA Moleküle in der Ausgangsmischung (4200) und nach der Selektion (27) ergibt dies eine Anreicherung der für M.Hae III-Calmodulin kodierenden DNA Moleküle von 153.

## Patentansprüche

1. Ein Verfahren zur Herstellung und Zuordnung von Nukleinsäuren und der **dadurch** kodierten Polypeptide, das folgende Schritte umfasst:
a) die Kompartimentierung von Nukleinsäuren zusammen mit einer *in vitro* Transkriptions-Translations-Mischung in einer Wasser-in-Öl-Emulsion,
b) die *in vitro* Expression der durch die Nukleinsäuren kodierten Fusionspolypeptide in den Mikrokompartimenten der Wasser-in-Öl-Emulsion, wobei jede Nukleinsäure an das Fusionspolypeptid gebunden wird, für das es kodiert,
wobei die Fusionspolypeptide jeweils mindestens einen konstanten Peptidanteil I und mindestens einen variablen Peptidanteil II umfassen und die Fusionspolypeptide über den Peptidanteil I mit der das Fusionspolypeptid kodierenden Nukleinsäure in Schritt b) kovalent verbunden werden, und
wobei die Anzahl der derart gebundenen Fusionspolypeptide pro Nukleinsäure eine definierbare ganze Zahl ist.

2. Das Verfahren gemäß Anspruch 1, wobei das Verfahren zusätzlich den folgenden Schritt umfasst:
c) das Extrahieren der in Schritt b) hergestellten Fusionspolypeptid-Nukleinsäure-Komplexe aus der Wasser-in-Öl-Emulsion.

3. Das Verfahren gemäß Anspruch 1 oder 2, wobei das Verfahren zusätzlich den folgenden Schritt umfasst:
d) das Auswählen von Fusionspolypeptid-Nukleinsäure-Komplexen mit gewünschten Eigenschaften.

4. Das Verfahren gemäß einem der Ansprüche 1 bis 3, wobei das Verfahren zusätzlich den folgenden Schritt umfasst:
e) das Amplifizieren der ausgewählten Nukleinsäuremoleküle.

5. Das Verfahren gemäß einem der Ansprüche 1 bis 4, wobei das Verfahren zusätzlich den folgenden Schritt umfasst:
f) das zufällige oder gerichtete Mutieren von einem oder mehreren Nukleotid(en) während oder nach dem Amplifizieren von Schritt e).

6. Das Verfahren gemäß einem der Ansprüche 1 bis 5, wobei das Verfahren zusätzlich den folgenden Schritt umfasst:
g) die ein- oder mehrfache Wiederholung eines der in den Ansprüchen 1 bis 5 beschriebenen Verfahren.

7. Das Verfahren gemäß einem der Ansprüche 1 bis 6, wobei die Nukleinsäuren rRNA, mRNA oder DNA sind.

8. Das Verfahren gemäß einem der Ansprüche 1 bis 7, wobei die Nukleinsäuren DNA sind.

9. Das Verfahren gemäß einem der Ansprüche 1 bis 7, wobei die Nukleinsäuren doppelsträngige DNA, vorzugsweise doppelsträngige lineare DNA sind.

10. Das Verfahren gemäß einem der Ansprüche 1 bis 9, wobei die Nukleinsäuren chemisch modifizierte DNA sind.

11. Das Verfahren gemäss einem der Ansprüche 1 bis 10, wobei jedes Mikrokompartiment der Wasser-in-Öl-Emulsion nicht mehr als eine Nukleinsäure umfasst.

12. Das Verfahren gemäss einem der Ansprüche, 1 bis 11, wobei die in der Wasser-in-Öl-Emulsion hergestellten Mikrokompartimente einen mittleren Durchmesser von 1 µm bis 2 µm aufweisen.

13. Das Verfahren gemäss einem der Ansprüche 1 bis 12, wobei jeweils ein Peptidanteil I mit einem Nukleinsäuremolekül kovalent verbunden ist.

14. Das Verfahren gemäss einem der Ansprüche 1 bis 13, wobei der konstante Peptidanteil I des Fusionspolypeptids eine (Cytosin-5)-Methyltransferase ist.

15. Das Verfahren gemäss Ansprüche 14, wobei die Methyltransferase aus der Gruppe ausgewählt ist, die aus M.Hae III, M.Hha I, M.Hpa I, M.Msp I, und Alu I besteht.

16. Das Verfahren gemäss Anspruch 15, wobei die Methyltransferase Hae III Methyltransferase aus *Haemophilus aegypticus* ist.

17. Das Verfahren gemäss Anspruch 16, wobei die modifizierte Nukleinsäure die Sequenz 5'-GGFC-3' umfasst und F 5-Fluorodeoxycytidin ist.

18. Eine Verwendung mindestens einer (Cytosin-5)-Methyltransferase in einem Verfahren gemäß einem der Ansprüche 1 bis 17.

19. Eine Verwendung von Fusionspolypeptiden oder damit kovalent gebundenen Nukleinsäure-Fusionspolypeptid-Komplexen in einem Verfahren gemäß einem der Ansprüche 1 bis 17, die jeweils einen konstanten Peptidanteil I und einen variablen Peptidanteil II umfassen, wobei die Fusionspolypeptide über den Peptidanteil I mit der das Fusionspolypeptid kodierenden Nukleinsäure kovalent verbunden werden oder verbunden sind, und wobei die Anzahl der derart gebundenen Fusionspolypeptide pro Nukleinsäure eine definierbare ganze Zahl ist.

## Claims

1. A method for the production and allocation of nucleic acids and the polypeptides coded by these, comprising the following steps:
a) the compartmentalisation of nucleic acids together with an *in vitro* transcription-translation mixture in a water-in-oil emulsion,
b) the *in vitro* expression of the fusion polypeptides coded by said nucleic acids in the microcompartments of the water-in-oil emulsion, whereby each nucleic acid is bonded to the fusion polypeptide it codes for,
wherein each of the fusion polypeptides comprises at least one constant peptide part I and at least one variable peptide part II, and wherein the fusion polypeptides are covalently bonded to the nucleic acid coding for said fusion polypeptide in step b), and wherein the number of the fusion polypeptides per nucleic acid bonded in this manner is a definable integer.

2. The method according to claim 1, wherein the method additionally comprises the following step:
c) the extraction of the fusion polypeptide-nucleic acid complexes prepared in step b) from the water-in-oil emulsion.

3. The method according to claim 1 or 2, wherein the method additionally comprises the following step:
d) the selection of fusion polypeptide-nucleic acid complexes with desired properties.

4. The method according to one of claims 1 to 3, wherein the method additionally comprises the following step:
e) the amplification of the selected nucleic acid molecule.

5. The method according to one of claims 1 to 4, wherein the method additionally comprises the following step:
f) the random or directed mutation of one or more nucleotide(s) during or after the amplification of step e).

6. The method according to one of claims 1 to 5, wherein the method additionally comprises the following step:
g) the repetition of one of the methods described in claims 1 to 5 once or several times.

7. The method according to one of claims 1 to 6, wherein the nucleic acids are rRNA, mRNA or DNA.

8. The method according to one of claims 1 to 7, wherein the nucleic acid is DNA.

9. The method according to one of claims 1 to 7, wherein the nucleic acids are double-stranded DNA, preferably double-stranded linear DNA.

10. The method according to one of claims 1 to 9, wherein the nucleic acids are chemically modified DNA.

11. The method according to one of claims 1 to 10, wherein each microcompartment of the water-in-oil emulsion does not comprise more than one nucleic acid.

12. The method according to one of claims 1 to 11, wherein the microcompartments prepared in the water-in-oil emulsion have an average diameter of 1 µm to 2 µm.

13. The method according to one of claims 1 to 12, wherein one peptide part I is covalently bonded to one nucleic acid molecule each.

14. The method according to one of claims 1 to 13, wherein the constant peptide part I of the fusion polypeptide is a (cytosine-5-)-methyl transferase.

15. The method according to claim 14, wherein the methyl transferase is selected from the group consisting of M.Hae III, M.Hha I, M.Hpa I, M.Msp I and Alu I.

16. The method according to claim 15, wherein the methyl transferase is Hae III methyl transferase from *haemophilus aegypticus.*

17. The method according to claim 16, wherein the modified nucleic acid comprises the sequence 5'-GGFC-3' and F is 5-fluorodeoxycytidine.

18. A use of at least one (cytosine-5-)-methyl transferase in a method according to one of claims 1 to 17.

19. Use of fusion polypeptides or covalently bonded nucleic acid-fusion polypeptide complexes in a method according to one of claims 1 to 17, that comprise a constant peptide part I and a variable peptide part II each, wherein the fusion polypeptides are or will be covalently bonded to the nucleic acid coding said fusion polypeptide by the peptide part I, and wherein the number of fusion polypeptides per nucleic acid bonded in this manner is a definable integer.

## Revendications

1. Procédé en vue de la fabrication et de l'attribution d'acides nucléiques et des polypeptides ainsi codés, qui comprend les étapes suivantes:
a) la compartimentation des acides nucléiques conjointement à un mélange translation-transcription *in vitro* dans une émulsion eau-dans-huile,
b) l'expression *in vitro* des polypeptides de fusion codés par les acides nucléiques dans les micro compartiments de l'émulsion eau-dans-huile, chaque acide nucléique étant lié au polypeptide de fusion, pour lequel il code,
les polypeptides de fusion comprenant à chaque fois au moins une portion de peptide I constante et au moins une partie de peptide II variable et les polypeptides de fusion étant liés dans l'étape b) d'une manière covalente, par l'intermédiaire de la portion de peptide I, avec l'acide nucléique codant le polypeptide de fusion, et le nombre de polypeptides de fusion ainsi liés par acide nucléique étant un nombre entier définissable.

2. Procédé selon la revendication 1, le procédé comprenant en sus l'étape suivante:
c) l'extraction du complexe acide nucléique-polypeptide de fusion fabriqué dans l'étape b) à partir de l'émulsion eau-dans-huile.

3. Procédé selon la revendication 1 ou 2, le procédé comprenant en sus l'étape suivante:
d) la sélection de complexes acide-nucléique-polypeptide de fusion ayant les propriétés souhaitées.

4. Procédé selon l'une quelconque des revendications 1 à 3, le procédé comprenant en sus l'étape suivante:
e) l'amplification des molécules d'acides nucléiques sélectionnées.

5. Procédé selon l'une quelconque des revendications 1 à 4, le procédé comprenant en sus l'étape suivante:
f) la mutation aléatoire ou orientée d'un ou de plusieurs nucléotide(s) pendant ou après l'amplification de l'étape e).

6. Procédé selon l'une quelconque des revendications 1 à 5, le procédé comprenant en sus l'étape suivante:
g) la répétition à une ou à plusieurs reprises d'un des procédés décrits dans les revendications 1 à 5.

7. Procédé selon l'une quelconque des revendications 1 à 6, les acides nucléiques étant ARNr, ARNm ou ADN.

8. Procédé selon l'une quelconque des revendications 1 à 7, les acides nucléiques étant ADN.

9. Procédé selon l'une quelconque des revendications 1 à 7, les acides nucléiques étant ADN à double hélice, préférablement ADN linéaire à double hélice.

10. Procédé selon l'une quelconque des revendications 1 à 9, les acides nucléiques étant ADN modifié du point de vue chimique.

11. Procédé selon l'une quelconque des revendications 1 à 10, chaque micro compartiment de l'émulsion eau-dans-huile ne comprenant pas plus d'un acide nucléique.

12. Procédé selon l'une quelconque des revendications 1 à 11, les micro compartiments fabriqués dans l'émulsion eau-dans-huile présentant un diamètre moyen de 1 µm à 2 µm.

13. Procédé selon l'une quelconque des revendications 1 à 12, une portion de peptide I étant à chaque fois liée d'une manière covalente à une molécule d'acide nucléique.

14. Procédé selon l'une quelconque des revendications 1 à 13, la portion de peptide I constante du polypeptide de fusion étant la (cytosine-5)-méthyltransférase.

15. Procédé selon la revendication 14, la méthyltransférase étant sélectionnée parmi le groupe qui se compose de M.Hae III, de M.Hha I, de M.Hpa 1, de M.Msp I, et de Alu I.

16. Procédé selon la revendication 15, la méthyltransférase Hae III étant la méthyltransférase provenant de l'Haemophilus aegypticus.

17. Procédé selon la revendication 16, l'acide nucléique modifié comprenant la séquence 5'-GGFC-3' et F étant la 5-Fluorodéoxycytidine.

18. Utilisation d'au moins une (cytosine-5)-méthyltransférase dans un procédé selon l'une quelconque des revendications 1 à 17.

19. Utilisation de polypeptides de fusion ou de complexes polypeptide de fusion-acide nucléique liés ainsi d'une manière covalente, dans un procédé selon l'une quelconque des revendications 1 à 17, qui comprennent à chaque fois une portion de peptide 1 constante et une portion de peptide II variable, les polypeptides de fusion étant liés d'une manière covalente par l'intermédiaire de la portion de peptide I avec l'acide nucléique codant le polypeptide de fusion ou étant liés et le nombre de polypeptides de fusion ainsi liés par acide nucléique étant un nombre entier définissable.
